# EUROPÄISCHE PATENTSCHRIFT

(11) **EP 2 603 194 B1**
(45) Veröffentlichungstag und Bekanntmachung des Hinweises auf die Patenterteilung: **09.10.2019**
(21) Anmeldenummer: 11743529.7
(22) Anmeldetag: 11.08.2011
(51) Int. Cl.: A61K 8/04, A61K 8/49, A61Q 15/00

(54) **STABILISIERTE W/O-EMULSIONEN**
STABILIZED W/O EMULSIONS
ÉMULSIONS EAU-HUILE STABILISÉES

(30) Priorität: 13.08.2010 DE 102010034389
(43) Veröffentlichungstag der Anmeldung: 19.06.2013
(73) Patentinhaber: Beiersdorf AG, 20253 Hamburg (DE)
(72) Erfinder: MIERTSCH, Heike, 22529 Hamburg (DE); URBAN, Michael, 22523 Hamburg (DE); HÜTTL, Matthias, 22769 Hamburg (DE); MÜHLEN, Nicole, 22946 Trittau (DE)
(86) Internationale Anmeldenummer: PCT/EP2011/063812
(87) Internationale Veröffentlichungsnummer: WO 2012/020075

(56) Entgegenhaltungen:
- WO-A1-96/18374
- WO-A1-98/17238
- WO-A1-2006/125582
- WO-A2-2004/091555
- WO-A2-2009/015857
- WO-A2-2009/126854
- DE-A1- 4 440 499
- US-A- 3 705 855
- US-A- 4 072 742
- US-A- 4 350 605
- US-A- 4 695 451
- US-A1- 2007 292 373

## Beschreibung

Die Erfindung umfasst kosmetische oder dermatologische Antitranspirantzubereitungen gemäss Anspruch 1 auf Basis von Wasser-in-ÖI Emulsionen. Die Zubereitungen enthalten mindestens einen W/O-Emulgator und ein oder mehrere Sorbitan-mono-, -di-, -tri- oder -tetraoleate und sind darüber hinaus frei von Polyglycerin-Emulgatoren. Die Emulgatorkombination führt zu einer erhöhten Stabilität der Emulsion.

Aus dem Stand der Technik sind vielfach kosmetische oder dermatologische Zubereitungen auf Basis von Wasser-in-ÖI Emulsionen bekannt.

EP 1 192 935 A2 offenbart W/O-Emulsionen umfassend Polyether wie PEG-45 (dodecyl glycol) copolymer und PEG-22 (dodecyl glycol) copolymer.

WO 2004/ 030 641 A1 offenbart als Aerosol versprühbare W/O-Emulsionen. Die im Stand der Technik als bevorzugt genannten Emulgatoren der Sorbitansesquioleate werden als problematisch aufgrund ihrer Oxidationsneigung und Destabilisierung der Emulsion aufgeführt. Als bevorzugte Ölkomponenten kosmetischer W/O-Emulsionen werden Listen verschiedenster Lipide aufgeführt, die neben Octyldodecanol immer auch Dicaprylylcarbonate nebeneinander aufführen.

Die WO 2003/ 039 508 A1 offenbart dünnflüssige und sprühbare W/O-Emulsionen umfassend drei verschiedenen Emulgatoren, von W/S- über W/O- zu O/W-Emulgatoren, wobei Polyglycerin-Emulgatoren als W/O-Emulgatoren bevorzugt sind.

EP 1 440 685 A1 offenbart kosmetische Zubereitungen auf W/O-Emulsionsbasis umfassend ebenfalls verschiedene Emulgatoren, wobei Sorbitantrioleat als einer von mehreren Stoffen aufgeführt ist.

Die WO 96/ 24 326 A1 offenbart Antitranspirant (AT)-Aerosolzubereitungen in Form von W/O-Emulsionen umfassend Aluminiumsalz, flüchtiges Silikonöl und Silikonemulgator in einer Aluminiumdose.

Die WO 2006/ 050 776 A1 beschreibt AT-Aerosolzubereitungen in W/O-Form umfassend Silikonemulgator, Silikonöl und C12-15 Alkyl Benzoate in Aluminiumdosen mit bestimmten Innenschutzlacken. Als optionale W/O-Emulgatoren werden u.a. Sorbitol Mono- und Diester der Myristin-, Palmitin- und Stearinsäure benannt.

Die WO 2006/ 133 725 A1 beschreibt AT-Aerosolzubereitungen in W/O-Form umfassend einen Polyglycerin-Emulgator, Isododecane und Isopropylpalmitat in Aluminiumdosen mit einer Zerstäubereinrichtung auf Basis einer Kapillarröhre (Truspray). Als optionale W/O-Emulgatoren werden neben anderen Emulgatoren Sorbitan Monolaurate und Sorbitan Monooleate benannt.

Die WO 2007/ 085 299 A1 beschreibt Aerosolzubereitungen in W/O-Form beinhaltend ein wasserlösliches Aluminiumsalz und ein Feuchthaltemittel.

Die EP 1 328 246 A2 beschreibt klare, schweißhemmende W/Si-Gele mit einem Refraktionsindex kleiner 1,42. Als Emulgatoren und/oder Co-Emulgatoren werden u.a. Sorbitanester und ethoxylierte Sorbitanester, wie z.B. PEG-20 Sorbitan Isostearate, Sorbitan Monolaurate, Polysorbate-20, Polysorbate-40, Polysorbate-60 und Polysorbate-80 benannt.

Die EP 1 286 650 A2 beschreibt hochviskose Emulsionen vom Typ W/Si mit einem hohen Gehalt an innerer Phase und angepassten Refraktionsindizes.

Die EP 0 998 909 A1 beschreibt AT-Zubereitungen in W/O-Form umfassend ein Alaunsalz, welches in Wasser gelöst vorliegt und in Form von Aerosolen dargereicht werden kann.

Die EP 0 605 483 A1 beschreibt ein Dosier-Aerosol mit einem Treibmittel bestehend aus Isobutan und Sorbitantrioleat.

Die EP 1 673 060 A1 beschreibt AT-Zubereitungen in W/O Form mit hydrophob modifizierten Silikaten für die Verwendung in Aerosolen.

Die WO 2001/ 018 145 A2 beschreibt Zubereitungen in Form von O/W- oder W/O-Emulsionen, die bei Ausbringung aus Aerosol-Packmitteln Tröpfchen mit unipolaren Ladungszuständen ermöglichen.

Die DE 199 08 210 A1 beschreibt Arzneimittel-Aerosole für die pulmonale Anwendung enthaltend Arzneimittel in suspendierter Form, 0,001% - 5% einer oder mehrerer oberflächenaktiven Substanz ausgewählt aus Sorbitan-Trifettsäureestern und mind. 90 % Treibmittel, wobei dieses zu mind. 75 % aus Propan besteht.

Die WO 2006/ 125 582 A1 beschreibt schweißhemmende W/O-Zubereitungen in Aerosol-Form, die einen Wirkstoff mit haarwuchshemmender Wirkung umfassen.

Die WO 2006/ 063 726 A1 beschreibt AT- bzw. Deo-Mittel-haltige W/O-Emulsionen und Ausführungsformen dazugehöriger Ventile.

Die EP 1 557 153 A1 beschreibt dünnflüssige W/O- oder W/Si-Emulsionen, die ohne Zugabe von O/W-Emulgatoren formuliert werden. Als mögliche Emulgatoren werden u.a. Sorbitanstearat, Sorbitanoleat und Sorbitanisostearat genannt. Der Lipidgehalt der Gesamtformulierung liegt über 25 Gew.%, bezogen auf die Gesamtformulierung inkl. Treibgas.

Die WO 92/ 18 227 A2 beschreibt stabile multiple Emulsionen (W/O/W und O/W/O), welche einen hydrophoben und einen hydrophilen Emulgator beinhalten. Als hydrophobe Emulgatoren werden u.a. Sorbitanoleat und Sorbitanmonostearat genannt.

Die WO 98/ 15 254 A1 beschreibt W/O Mikroemulsionen in Form eines Gels, in denen die Wasserphase u.a. einen oder mehrere Sorbitanester als W/O-Emulgatoren beinhaltet.

Die WO 98/ 17 238 A1 beschreibt Deo- oder AT-Stifte, die u.a. einen oder mehrere W/O-Emulgatoren beinhalten und diese Emulgatoren u.a. aus Sorbitanestern von Polyolen gewählt werden kann.

Die DE 102 13 957 A1 beschreibt ein Verfahren zur Herstellung von vernetzten Mikroemulsionsgelen oder vernetzten O/W-Emulsionen beinhaltend u.a. mindestens einen O/W-Emulgator und ggf. einen W/O-Emulgator. Als ein möglicher O/W-Emulgator wird Sorbitanisostearat genannt.

Die DE 102 13 955 A1 beschreibt ein Verfahren zur Herstellung von niedrigviskosen, insbesondere versprühbaren, phospholipidhaltigen O/W-Emulsionen beinhaltend u.a. mindestens einen O/W-Emulgator und ggf. einen W/O-Emulgator. Als ein möglicher O/W-Emulgator wird Sorbitanisostearat genannt.

Die DE 195 48 013 A1 beschreibt O/W-Emulsionen, insbesondere O/W-Mikroemulsionen, O/W/O-Emulsionen oder O/W/O'-Emulsionen, enthaltend u.a. einen Emulgator, der aus der Gruppe der Sorbitanester und Saccharoseester gewählt werden kann. Bevorzugt benannt werden hier u.a. Sorbitanstearat, Sorbitanoleat, Glycerylsorbitanstearat.

Die WO 2005/ 117 825 A1 beschreibt W/O-Emulsionen, die 2-Phenylethylbenzoat enthalten. Als W/O-Emulgatoren werden hier u.a. Sorbitanoleat, Sorbitanstearat, Sorbitanisostearat benannt.

Die DE 102 342 57 A1 beschreibt Substrate (Tücher), die sich bei Kontakt mit Wasser erwärmen, sowie das Verfahren zur Herstellung dieser Substrate und ihre Verwendung. Sorbitantrioleat wird als ein möglicher Rohstoff genannt.

Die WO 2004/ 000 243 A1 beschreibt schäumbare kosmetische Formulierungen, die u.a. als Emulgator PEG-40-Sorbitanlanolat enthalten und als Co-Emulgator Sorbitanstearat in Verbindung mit einem oder mehreren Fettalkoholen enthalten können.

Die DE 196 43 238 A1 beschreibt Deo- oder AT-Stifte, die u.a. einen oder mehrere W/O-Emulgatoren beinhalten und diese Emulgatoren u.a. aus Sorbitanestern von Polyolen gewählt werden können.

Die WO 03/ 082 223 A2 beschreibt O/W-Emulsionen, die zusätzlich zum O/W-Emulgator W/O-Emulgatoren enthalten können. Als O/W-Emulgator wird u.a. Sorbitanisostearat genannt.

Die DE 100 63 660 A1 beschreibt O/W-Emulsionen, die u.a. 0,1 bis zu 1,5 Gew.-% eines oder mehrerer Monoester von Sorbitol, gewählt aus der Gruppe Sorbitanstearat, Sorbitandistearat, Sorbitanisostearat, Sorbitanoleat, PEG-40-Sorbitanperoleat, PEG-40-Sorbitanperisostearat, Sorbitansesquioleat enthalten können.

Die DE 100 63 658 A1 beschreibt W/O/W-Emulsionen, die u.a. 0,1 bis zu 1,5 Gew.-% einen oder mehrere Monoester von Sorbitol, gewählt aus der Gruppe Sorbitanstearat, Sorbitandistearat, Sorbitanisostearat, Sorbitanoleat, PEG-40-Sorbitanperoleat, PEG-40-Sorbitanperisostearat, Sorbitansesquioleat enthalten können.

Die EP 2 002 864 A1 beschreibt kosmetische Kapseln für kosmetische Inhaltsstoffe. Der Kapselhülle können u.a. folgende W/O-Emulgatoren zugesetzt werden: PEG-40 Sorbitanperisostearat, Sorbitanisostearat, Sorbitanstearat, PEG-40 Sorbitanperoleat, Sorbitanoleat.

Die EP 0 783 882 A2 und die EP 0 782 848 A2 beschreiben O/W-Emulsionen, insbesondere O/W-Mikroemulsionen, die einen in Ölkomponenten schwer löslichen UV Filter beinhalten. Als mögliche Emulgatoren für dieses System werden u.a. Sorbitanstearat, Sorbitanoleat, Glycerylsorbitanstearat genannt.

EP 0 783 881 A2 beschreibt O/W-Emulsionen, insbesondere O/W-Mikroemulsionen, die einen Emulgator enthalten, dessen Lipophilie abhängig vom pH-Wert oder der Temperatur ist. Als mögliche Emulgatoren für dieses System werden u.a. Sorbitanstearat, Sorbitanoleat, Glycerylsorbitanstearat genannt.

Die DE 10 2005 028 590 A1 beschreibt O/W-Emulsionen mit einem Gehalt von 5-25 Gew.-%, an Isotridecylisononanoat. Als mögliche Emulgatoren werden u.a. Polysorbate-20, Polysorbate-60, Polysorbate-65, Polysorbate-100, Sorbitansesquioleat, Sorbitanstearat, Sorbitanoleat, Sorbitanisostearat aufgeführt.

Die DE 103 44 888 A1 beschreibt ein Verfahren zur Herstellung von Emulsionen oder Gelcremes vom Typ Öl-in-Wasser. PEG-20 Sorbitanstearat, PEG-20 Sorbitanisostearat, PEG-20 Sorbitanoleat, Sorbitanisostearat, PEG-160-Sorbitantriisostearat werden u.a. als Emulgatoren genannt.

Die DE 101 39 582 A1 beschreibt W/O/W-Emulsionen, die u.a. Sorbitanstearat, Sorbitandistearat, Sorbitanisostearat, Sorbitanoleat, PEG- 40-Sorbitanperoleat, PEG-40-Sorbitanperisostearat, Sorbitansesquioleat enthalten können.

Die DE 101 39 580 A1 beschreibt O/W-Emulsionen, die u.a. Sorbitanstearat, Sorbitandistearat, Sorbitanisostearat, Sorbitanoleat, PEG- 40-Sorbitanperoleat, PEG-40-Sorbitanperisostearat, Sorbitansesquioleat enthalten können.

Die DE 100 63 659 A1 beschreibt ein Verfahren zur Herstellung sprühbarer Emulsionen, die über einen Zeitraum von mindestens 4 Minuten bei mindestens 2500 U/min mittels des Standard-Rotor-Stator-Prinzips homogenisiert werden. Als Emulgatoren können u.a. 0,1 bis zu 1,5 Gew.-% eines oder mehrerer Monoester von Sorbitol, gewählt aus der Gruppe Sorbitanstearat, Sorbitandistearat, Sorbitanisostearat, Sorbitanoleat, PEG-40-Sorbitanperoleat, PEG-40-Sorbitanperisostearat, Sorbitansesquioleat enthalten sein.

Die DE 196 42 090 A1 beschreibt Mikroemulsionen vom Typ Wasser-in-ÖI, in denen die Wasserphase u.a. einen oder mehrere Sorbitanester als W/O-Emulgatoren enthalten kann.

Die EP 1 216 682 A2, WO 02/ 074 258 A2, WO 02/ 074 257 A2, WO 02/ 074 253 A1, WO 02/ 074 264 A1 und WO 02/ 074 256 A2 beschreiben selbst-schäumende oder schaumförmige O/W-Zubereitungen, die zwei Emulgatoren und einen Coemulgator beinhalten. Zusätzlich zu den dann drei verschiedenen Emulgatoren können bis zu 5 Gew.% weitere Emulgatoren, gewählt aus der Gruppe der hydrophilen Emulgatoren gewählt werden. Neben anderen Emulgatoren sind hier insbesondere Mono-, Di-, Trifettsäureester des Sorbitols genannt.

DE 695 35 310 T2 offenbart stabile Wasser-in-ÖI Emulsionssysteme, die zwingend ein Organopolysiloxan-Elastomer zur Stabilisierung enthalten.

US 6 403 067 B1 beschreibt stabile Emulsionen in Gelform, insbesondere Wasser-in-Silikon Emulsionen. Die Emulsionen haben einen geringen Wassergehalt von maximal 15%.

US 6 171 581 B1 beschreibt feste Deodorant- und Antitranspirantzubereitungen in Form von Wasser-in-ÖI Emulsionen. Die festen Zubereitungen umfassen einen Silikon-Elastomer.

US 3 705 855 offenbart Treibgas-haltige Emulsionen, die nach dem Applizieren und der Treibgasverflüchtigung umschlagen, d.h. von Wasser-in-ÖI (W/O) in Öl-in-Wasser (O/W) Emulsionen umschlagen.

Sorbitane bilden eine Sammelbezeichnung für 4-wertige Alkohole, die durch Entzug von 1 Mol Wasser aus d-Glucitol (Sorbit) durch Erhitzen unter dem katalytischen Einfluss von Säuren entstehen. So entstehen durch Veresterung mit Fettsäuren (Laurin-, Öl-, Palmitin- oder Stearinsäure) entsprechende Sorbitanfettsäureester (Sorbitanester), die als nichtionische Tenside bekannt sind. Durch Verknüpfung von Sorbitanen mit Polyoxyethylen (ethoxylierte Sorbitanester) können die hydrophilen Eigenschaften der Sorbitanfettsäureester gesteigert werden. Diese Verbindungen werden auch als Polysorbate (früher Sorbimacrogol) bezeichnet. Bereits 0,25 Gew.% in der Zubereitung ohne Treibmittel an eingesetzten Polysorbaten, wie beispielsweise Polysorbate-65 oder Polysorbate-20 können sich negativ auf die Stabilität der enthaltenen W/O-Zubereitung auswirken. Bei höheren Gehalten an Polysorbaten kann es ferner auch zur Ausbildung von unerwünschten O/W-Emulsionen kommen.

Aufgabe ist es daher, u.a. die Verwendung von ethoxilierten Verbindungen weitest möglich zu reduzieren, d.h. den Einsatz ethoxilierter Sorbitanfettsäureester zu vermindern bzw. ganz zu vermeiden.

Sorbitantrioleat, CAS: 26266-58-0, ist ein W/O-Emulgator mit einem HLB-Wert von 1,8 nachfolgender Struktur:

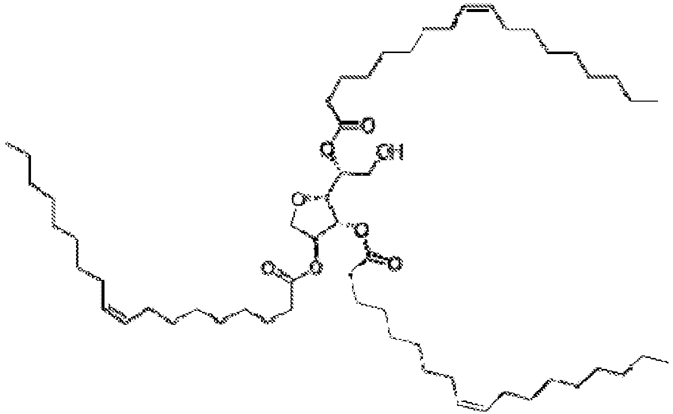

Sorbitantrioleat wird in verschiedensten Arzneimitteln u.a. auch als Wirkverstärker eingesetzt. Im Handel erhältliches Sorbitantrioleat ist beispielsweise das Span® 85.
Andere bekannte Sorbitanfettsäureester sind beispielsweise das Sorbitanmonooleat (Span®80), Sorbitantristearat (Span®65) oder das Sorbitanmonolaurat (Span®20).

Kosmetische Zubereitungen auf Basis einer Wasser-in-ÖI Emulsion, insbesondere dünnflüssige und sprühbare Emulsionen, zeigen häufig Instabilitäten. Zum Einen wird nach längerer Lagerung oder thermischer Belastung eine Separation beobachtet, es bildet sich freies Wasser, oder zum Anderen wird unter Treibmittel die Bildung von gelartigen Rückständen beobachtet. Beispielsweise wird nach längeren Lagerzeiten unter Treibmittel bei Stand der Technik Antitranspirantsprays auf W/O-Emulsionsbasis die Bildung eines gelartigen Rückstandes beobachtet, der nur sehr schwer durch Aufschütteln der Dose reversibel ist. Dieser Rückstand bedingt ungleichmäßigen Produktaustrag, welcher vom Anwender als verstärktes Weißeln wahrgenommen werden kann. Ferner kann dies zur Verstopfung des Sprayventils und/oder Sprühkopfes bzw. Düse führen.
Dünnflüssig, sprühbare W/O-Emulsionen des Standes der Technik, wie sie beispielsweise in der Druckschrift EP 1 474 099 A1 beschrieben sind, umfassen häufig komplexe Emulgatorkombinationen. Diese Emulgatoren erfordern bei der Herstellung der Endzubereitungen im Produktionsmaßstab eine zeitlich und logistisch aufwändige Rohstoffvorbehandlung wie z.B. deren Aufschmelzung in Wärmeschränken. Wiederholte thermische Belastung von Vorratsgebinden infolge dieser Vorbehandlung kann zu einer Beeinträchtigung der Emulgatoreigenschaften führen, welche in nicht berechenbaren galenischen Schwierigkeiten münden und zu den aufgeführten Problemen führen können. Problematisch gestalten sich auch Emulsionen, die separieren und somit dann freies Wasser in einer Spraydose vorliegt, welches dann zur Korrosion der Dose oder Ventile bzw. Ventilteller führen kann.
Eine Ursache dieser Probleme sind vermutlich Schwankungen in der Qualität der eingesetzten Emulgator-Rohstoffe.

Beide Probleme, also sowohl die nicht qualifizierbaren Qualitätsprobleme des kommerziell verfügbaren Emulgator-Rohstoffes, wie auch die Einschränkungen durch notwendige Vorbehandlungen der Emulgatoren, gilt es zu vermeiden bzw. zu verbessern.

Weiterhin wäre es wünschenswert, weniger komplexe Emulgatorkombinationen bereit zu stellen, um thermisch und zeitlich stabile Wasser-in-ÖI Emulsionen für kosmetische Zubereitungen zur Verfügung zu haben.
Weiterhin ist es die Aufgabe der vorliegenden Erfindung kosmetische oder dermatologische Zubereitungen auf W/O-Emulsionsbasis bereit zu stellen, die wenig oder keine Bodensätze, Rückstände oder unansehnliche Flecken auf der Haut oder Kleidung verursachen (Low-Residue-Eigenschaften).

Sprühgüter in Form von W/O-Formulierungen erfordern zur Erzeugung von Aerosolen vorwiegend lipophile Treibgase, da sich diese in der äußeren Phase der Emulsion lösen. Treibmittel, die sich hauptsächlich in der inneren Phase einer solchen Zubereitung lösen, erzeugen nach Verdampfen aus der inneren Phase im Allgemeinen einen Schaum. Wünschenswert ist es Zubereitungen zur Verfügung zu stellen, die nach der Applikation nicht aufschäumen, da dies die Sichtbarkeit und damit die Wahrnehmbarkeit von Rückständen erhöht.

Die in wasserhaltigen Deodorant- oder Antitranspirantzubereitungen enthaltenen Wirk-, Hilfs- und Zusatzstoffe können die Korrosion von Metallverpackungen fördern. Um den Kontakt mit dem korrosionsgefährdeten Material zu minimieren, sollten diese Stoffe bevorzugt in der inneren Phase der Wirkstofflösung formuliert werden. Nichtsdestotrotz ist es üblich und in der Regel notwendig, die eingesetzten Dosen von innen zu lackieren. Physikalische Instabilitäten der Wirkstofflösung wie z.B. Wasserabscheidungen mit Gehalten an korrosiven Substanzen können sich negativ auf das Packmittel sowie die Produktleistung auswirken.

Weiterhin wünschenswert ist es daher, Zubereitungen, insbesondere Deodorant- oder Antitranspirantzubereitungen, zur Verfügung zu stellen, die über eine gute Sprühfähigkeit verfügen, eine unproblematische Abfüllung als Aerosol ermöglichen, gute Kompatibilität mit lipophilen Treibgasen zeigen und eine gute Kompatibilität und Stabilität mit Antitranspirantwirkstoffen zeigen.

Insbesondere sollten sprühbare Antitranspirantzubereitungen zur Verfügung gestellt werden, die eine Verbesserung der Aufschüttelbarkeit der Gesamtzubereitung ermöglichen.

Unter Aufschüttelbarkeit im Sinne der Erfindung ist die Erreichung einer augenscheinlich vollständig homogenen Durchmischung von Treibgas und Wirkstofflösung zu verstehen. Das Verhalten von Wirkstofflösungen unter Treibgas wird üblicherweise unter Verwendung sogenannter Glas-Aerosole ermittelt.

Gelöst werden die geschilderten Probleme durch eine kosmetische oder dermatologische Antitranspirantzubereitungen entsprechend Anspruch 1 basierend auf einer Wasser-in-ÖI Emulsion umfassend mindestens einen W/O-Emulgator und ein oder mehrere Sorbitan-mono-, -di-, -tri- und/oder -tetraoleate.

Die Zubereitung ist frei von Polyglycerin-Emulgatoren und frei von Organopolysiloxan-Elastomeren.

Der Einsatz ethoxilierter Sorbitanfettsäureester, insbesondere Polysorbate, wird erfindungsgemäß reduziert bzw. verzichtet. Neben dem Verzicht auf Polyglycerin-Emulgatoren ist es erfindungsgemäß daher vorteilhaft, auch auf Polysorbate, insbesondere auf Polysorbate 65, zu verzichten.

Bevorzugt sind daher die Anteile an ethoxilierten Sorbitansäureestern, insbesondere Polysorbaten im Bereich von weniger als 0,25 Gew.%, insbesondere 0 Gew.%, bezogen auf die Gesamtmasse der Zubereitung ohne Treibgas, zu wählen.

Es ist bekannt, dass der in den Formulierungen des Standes der Technik enthaltene Polysorbatemulgator (z.B. Polysorbate 65) durch seine guten filmbildenden Eigenschaften zur Vermeidung von weißen Rückständen beiträgt.

Es stellte sich daher zusätzlich die Aufgabe bei Vermeidung von Polysorbatemulgatoren dennoch gleichwertige Anti-Weißeleigenschaften zu erzielen.

Dies ist überraschenderweise ebenfalls durch die erfindungsgemäßen Zubereitungen gelungen. Ein Verzicht auf Polysorbatemulgatoren ist somit möglich und bevorzugt.

Die erfindungsgemäßen Zubereitungen zeigen verminderte Weißeleigenschaften, insbesondere auf der Haut und Kleidung.

Die Zubereitungen umfassen in einer bevorzugten Ausführungsform nur einen der erfindungsgemäßen W/O-Emulgatoren und ein oder zwei Sorbitanoleate, bevorzugt ein Sorbitanoleat, wie beispielsweise Cetyl PEG/PPG-10/1 Dimethicone als W/O-Emulgatoren, und Sorbitantrioleat. D.h. in der erfindungsgemäßen Zubereitung sind bevorzugt keine weiteren Emulgatoren enthalten.
Es steht den Entwicklern kosmetischer oder dermatologischer Zubereitungen natürlich frei den Zubereitungen derartig geringe Mengen an Emulgatoren zu zusetzen um dennoch auch dann erfindungsgemäße Zubereitungen zu erhalten.

Ebenso können, müssen aber nicht, Tenside in der Zubereitung enthalten sein, ohne den erfindungsgemäßen Zubereitungsrahmen zu verlassen. Tenside können daher insbesondere bis zu 1 Gew.% eingearbeitet werden, bevorzugt 0,05-0,1% bezogen auf die Gesamtmasse der Zubereitung ohne Treibmittel.

Das Merkmal der Emulgatorfreiheit, mit Ausnahme der als erfindungsgemäß beanspruchten, umfasst daher Tenside nicht.

Tenside stellen einen Oberbegriff für waschaktive Substanzen dar. Emulgatoren unterscheiden sich demgegenüber in ihrer Wirkung der Stabilisierung von Emulsionen und ihres Verwendungszweckes.

Tenside sind Substanzen, die die Oberflächenspannung einer Flüssigkeit oder die Grenzflächenspannung zwischen zwei Phasen herabsetzen und die Bildung von Dispersionen ermöglichen oder unterstützen. Tenside bewirken, dass zwei eigentlich nicht miteinander mischbare Flüssigkeiten, wie zum Beispiel Öl und Wasser, dispergiert werden können.

Des Weiteren werden Tenside als amphiphile Stoffe beschrieben, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Emulgatoren bewirken, dass zwei nicht miteinander mischbare Flüssigkeiten (zum Beispiel Öl in Wasser) sich zu einer Emulsion vermengen können. Aufgrund des amphiphilen Charakters dringen sie mit ihrem fettlöslichen Teil in das Öl ein. Durch den hydrophilen Teil kann das nun durch Rühren entstandene Öltröpfchen in der wässrigen Umgebung dispergiert werden. Emulgatoren haben primär keinen waschaktiven, tensidischen Charakter.

Emulgatoren setzen die Grenzflächenspannung zwischen den beiden Phasen herab und erreichen neben der Verringerung der Grenzflächenarbeit auch eine Stabilisierung der gebildeten Emulsion. Sie stabilisieren die gebildete Emulsion durch Grenzflächenfilme sowie durch Ausbildung sterischer oder elektrischer Barrieren, wodurch das Zusammenfließen (Koaleszenz) der emulgierten Teilchen verhindert wird.

Damit Verbindungen als Emulgatoren wirksam sein können, müssen sie eine bestimmte Molekülstruktur aufweisen. Strukturelles Kennzeichen solcher Verbindungen ist ihr amphiphiler Molekülaufbau. Das Molekül einer solchen Verbindung besitzt wenigstens eine Gruppe mit Affinität zu Substanzen starker Polarität (polare Gruppe) und wenigstens eine Gruppe mit Affinität zu unpolaren Substanzen (apolare Gruppe).
Insbesondere wird erfindungsgemäß unterschieden zwischen nicht-ionisch, anionisch und kationischen Emulgatoren. Die für die Ausbildung der erfindungsgemäßen Emulsion relevanten Emulgatoren, der oder die W/O-Emulgatoren sowie die Sorbitanoleate, sind allesamt nicht-ionisch. Auf den Zusatz an kationischen und/oder anionischen Emulgatoren kann daher bevorzugt verzichtet werden. Unter nicht-ionischen Emulgatoren werden grenzflächenaktive Substanzen verstanden, die in wässriger Lösung keine Ionen bilden. Die Hydrophilie solcher nicht-ionischer Emulgatoren wird durch den Anteil der polaren Gruppen im Molekül erreicht.

Ein Kennzeichen für die Hydrophilie eines gegebenen Emulgators ist dessen HLB-Wert, der sich nach folgender Formel ergibt: HLB = 20 x (1- Mₗᵢₚₒₚₕᵢₗ/M), wobei Mₗᵢₚₒₚₕᵢₗ für die Molmasse des lipophilen Anteils im Emulgator und M für die Molmasse des gesamten Emulgators steht.

Im Allgemeinen gelten Emulgatoren mit einem HLB-Wert bis ca. 8 als W/O-Emulgatoren. O/W-Emulgatoren hingegen weisen HLB-Werte von größer 8 bis 15 auf. Substanzen mit HLB-Werten größer 15 werden häufig als Lösungsvermittler bezeichnet.

Im Stand der Technik werden W/O-Emulsionen zuweilen durch Zusatz von O/W-Emulgatoren (HLB > 8) stabilisiert, die die Funktion eines Co-Emulgators ausüben. Der Verzicht auf solche Substanzen verringert die Rohstoffkomplexität und vermeidet ein Umschlagen der Formulierung in den nicht gewünschten O/W-Bereich. Es ist erfindungsgemäß vorteilhaft auf den Zusatz von Emulgatoren mit einem HLB > 8 zu verzichten.

Tenside als waschaktive Substanzen und vor allem Anti-Staining, wie in der DE 10 2009 010 665 A1 beschrieben, sind anionische und/oder kationische Tenside und von daher auch von den erfindungsgemäßen nicht-ionischen Emulgatoren zu unterscheiden.

Erfindungsgemäß bevorzugt umfassen die kosmetischen oder dermatologischen Zubereitungen nur erfindungsgemäße Emulgatoren und der Anteil an anderen Emulgatoren ist unter 0,5 Gew.%, insbesondere 0 Gew.%, bezogen auf die Gesamtmasse der Zubereitung ohne Treibmittel zu wählen.

Die erfindungsgemäßen Zubereitungen werden bevorzugt dünnflüssig und sprühbar formuliert, damit sie als Aerosol applizierbar sind.

Auf den Zusatz an Verdickern, insbesondere Organopolysiloxan-Elastomer, kann daher verzichtet werden.

Ein Aerosol ist ein disperses System, bei dem ein Feststoff oder eine Flüssigkeit in einem Gas äußerst fein verteilt vorliegt. Das Aerosol wird in der Regel erst bei der Anwendung mit Hilfe eines geeigneten Sprühsystems durch Versprühen von Lösungen, Emulsionen oder Suspensionen selbst erzeugt, wozu beispielsweise Sprühdosen verwendet werden können, in denen ein verflüssigtes Druckgas als Treibgas dient. Beim Öffnen des Druckventils entweicht das Treibmittel-Zubereitungsgemisch durch eine feine Düse, das Treibmittel verdampft und hinterlässt das fein verteilte Sprühgut als Aerosol. Im anwendungstechnischen Sprachgebrauch wird der Begriff Aerosol vom Fachmann häufig auch im Sinne von Aerosol-Sprays verwendet, d.h. hier wird unter "Aerosol" nicht der reine Sprühnebel sondern eine Druckgasverpackung nebst Abgabe- bzw. Dosiervorrichtung und Füllgut verstanden.

Bei der Entwicklung von treibmittelhaltigen Formulierungen in Form von W/O-Emulsionen treten eine Reihe von besonderen Problemen auf, beispielsweise, dass die Ölphase sich als äußere Phase zu erheblichen Anteilen im Treibmittel lösen kann. Ferner muss bei der Formulierung beachtet werden, dass das Treibgas, welches im Allgemeinen erst beim Abfüllen der Zubereitung zugesetzt wird, einen Einfluss auf die physikalischen Eigenschaften des Produktes hat. So kann der Zusatz des Treibmittels z. B. eine Phasentrennung auslösen, so dass keine homogene Versprühung des Füllgutes möglich ist, oder die Viskosität des Produktes so verändern, dass es gar nicht erst sprühbar ist. Eine Verträglichkeitsprüfung der Wirkstofflösung mit dem Treibmittel ist daher unerlässlich.

Die erfindungsgemäßen Zubereitungen zeigen erstaunlicherweise eine verbesserte physikalische Stabilität - insbesondere auch unter Einfluss hoher Elektrolytkonzentrationen, insbesondere mit Antitranspirantwirkstoffen und lipophilen Treibmitteln.

Insbesondere können die erfindungsgemäßen Zubereitungen ohne thermische Belastungen bei der Vorbehandlung oder Endproduktion hergestellt werden, was ebenfalls zu einer Vereinfachung der Herstellverfahren als auch der Endzubereitungen führt.

Die Stabilität der erfindungsgemäßen dünnflüssigen und sprühbaren W/O-Emulsion wird dabei ohne den Zusatz an Organopolysiloxan-Elastomeren erreicht, da zudem ein solcher Zusatz die Dünnflüssigkeit und Sprühbarkeit verschlechtern würde.
Im Stand der Technik werden Verdicker und Silikon-Elastomere zur Stabilisierung von Emulsionen verwendeten, da aus thermodynamischen Gründen aus einem verdicktem System eine Phasentrennung sich langsamer vollziehen kann.
"Frei von" bedeutet erfindungsgemäß, dass der Anteil an diesen Stoffen, auf die erfindungsgemäß verzichtet werden kann, unterhalb derjenigen Menge liegt, beim dem der Zusatz an Stoffen, wie Organopolysiloxane und Polyglycerinemulgatoren, keine emulgierende und/oder verdickende Wirkung zeigt.
Bevorzugt bedeutet "frei von" ein Anteil von weniger als 0,1 Gew.%.

Basierend auf bekannten W/O-Formulierungen wurde nun überraschend eine Grundlage entwickelt, die ohne Polyglycerin-Rohstoffe, wie beispielsweise den Emulgator Dehymuls PGPH (Polyglyceryl-2 Dipolyhydroxystearate), auskommt und somit die Vorbehandlung der Rohstoffe hinfällig werden lässt.
Als Hauptemulgator dient in der erfindungsgemäßen Zubereitung ein W/O-Emulgator, der aus der Liste der bekannten Emulgatoren zu wählen ist, mit Ausnahme der Polyglycerin-Emulgatoren. Bevorzugt weisen der oder die W/O-Emulgatoren einen HLB-Wert von kleiner gleich 8 auf. Insbesondere ist der W/O-Emulgator mit einem HLB von gleich oder kleiner 5 zu wählen.
Besonders bevorzugt wird der W/O-Emulgator aus der Gruppe der W/Si-Emulgatoren gewählt. Bevorzugte W/O-Emulgatoren werden gewählt aus der Gruppe PEG/PPG-18/18 Dimethicone, Lauryl PEG/PPG-18/18 Dimethicone, Bis PEG/PPG 14/14 Dimethicone, Cetyl PEG/PPG-10/1 Dimethicone, Cocamidopropyl PG-Dimonium Chloride. Bevorzugt ist insbesondere der Emulgator Abil EM 90 (Cetyl PEG/PPG-10/1 Dimethicone).

Als erfindungswesentlich wird dieser W/O-Emulgator mit Sorbitan-mono-, -di-, -tri- oder - tetraoleat, insbesondere Sorbitantrioleat, als Co-Emulgator kombiniert.

In verschiedensten Untersuchungen zeigte sich, dass die Sorbitanoleate, insbesondere das Sorbitantrioleat, die Aufschüttelbarkeit der Emulsion im Treibmittel (Kohlenwasserstoffgemisch) verbessert. Ohne Zugabe der Sorbitanoleate, insbesondere von Sorbitantrioleat, verbindet sich die Emulsion nach dem Aufschütteln nicht komplett mit dem Treibmittel und es kommt zu Ausfällungen.

Ein Vergleichsversuch zweier Emulsionen (I: Beispiel E umfassend eine Emulgatorkombination der Druckschrift EP 1 474 099 A1; II: Beispiel 5) zeigte, dass sich bei I Rückstände nach 6-monatiger Lagerung bei Raumtemperatur unter lipophilem Treibmittel bildeten. Die erfindungsgemäße Zubereitung II hingegen zeigte auch nach 6 Monaten Lagerung unter lipophilem Treibgas keinerlei Rückstände. Zur Quantifizierung der Rückstandsbildung nach Lagerung wurden die Druckgasverpackungen kontrolliert entgast und anschließend geöffnet. Die in den geöffneten Dosen vorliegenden Füllgüter wurden zur besseren Darstellung auf Uhrgläser überführt. Dabei sind deutlich unerwünschte, gallertartige Bestandteile im Füllgut erkennbar. Das Füllgut erfindungsgemäßer Zubereitungen liegt hingegen vollständig dünnflüssig, ohne Beimengung von gallertartig-pastösen Bestandteilen vor. Zur weiteren Quantifizierung wurden die nach Entgasen der Druckgasverpackung vorliegenden Füllgüter vorsichtig dekantiert, um so die nichtflüssigen Bestandteile zu separieren. Von ursprünglich 29,3 g eingewogener, flüssiger Emulsion vor Begasung, ergibt sich nach Lagerung und Entgasung im Falle von I ein Dekantat von 20,4 g. Im Falle von II wurde ein Dekantat von 0,5 g ermittelt, was einem Dekantatanteil von 1,7 % entspricht.

Es ist somit erfindungsgemäß die Kombination umfassend mindestens einen W/O-Emulgator, ein oder mehrere Sorbitan-mono-, -di-, -tri- und/oder -tetraoleate und gegebenenfalls ein oder mehrere Guerbet-Alkohole oder -Ester in kosmetischen oder dermatologischen Zubereitungen unter Treibmitteln zur Verminderung des Dekantatanteils nach 6-monatiger Lagerung bei Raumtemperatur auf einen Anteil von weniger als 10%, insbesondere weniger als 5%, bezogen auf die ursprünglichen Einwaage der Zubereitung ohne Treibmittel, zu verwenden.

Durch den erfindungsgemäßen Verzicht von Polyglycerin-Emulgatoren lässt sich ein Gelieren der Emulsion unter Treibmittel verhindern.
Es wird so Verstopfungen und ungleichmäßigem Produktaustrag vorgebeugt.

Der oder die W/O-Emulgatoren werden bevorzugt zu einem Anteil von bis zu 5 Gew.%, insbesondere bis zu 2,5 Gew.%, bezogen auf die Gesamtmasse der Zubereitung ohne Treibmittel, gewählt.

Die Sorbitanoleate, insbesondere Sorbitantrioleate, werden bevorzugt zu einem Anteil von bis zu 4 Gew.%, insbesondere bis zu 2 Gew.%, bezogen auf die Gesamtmasse der Zubereitung ohne Treibmittel, gewählt.

Bevorzugt wird das Gewichtsverhältnis von W/O-Emulgator, insbesondere Cetyl PEG/PPG-10/1 Dimethicone, zu Sorbitanoleaten im Bereich von 1 bis 4 (W/O-Emulgator) zu 1 bis 4 (Sorbitanoleate), insbesondere 1 bis 2 (W/O-Emulgator) zu 1 bis 1,5 (Sorbitanoleate), gewählt. Die Beispiele 1 bis 7 bestätigen diese bevorzugten Verhältnisse eindrucksvoll.
Insbesondere ist ein Verhältnis von etwa 1,75 W/Si-Emulgator zu 1 Sorbitantrioleat bevorzugt zu wählen.

Die erfindungsgemäßen W/O-Emulsionen sind durch die Emulgatorkombination weniger störanfällig und stabiler.

Die verbesserte Stabilität zeichnet sich insbesondere dadurch aus, dass die Emulsion nach Lagerung bei niedrigen Temperaturen (6°C, -10°C) keine Instabilitäten wie z.B. Wasser- oder Ölabscheidungen aufweist. Des Weiteren zeigt sich die verbesserte Stabilität nach Lagerung bei wechselnden Temperaturen (z.B. Wechseltemperaturschrank).
Die Emulsion wird bei diesem Test für 6 Tage in einem Wechseltemperaturschrank gelagert, der alle 12 Stunden zwischen den Temperaturen -10°C und +40°C wechselt. Im Tagesabstand erfolgt visuell die Überprüfung der phys. Stabilität, diese sollte auch nach 6 Tagen noch einwandfrei sein, was bei den erfindungsgemäßen Zubereitungen im Gegensatz zu den aus den Stand der Technik bekannten Zubereitungen der Fall ist.
Durch Aufnahme rheologischer Kurven oder Bestimmung der Leitfähigkeit lässt sich diese Stabilitätssteigerung darstellen.

In der Praxis wird die Stabilität auch einfach durch in Augenscheinnahme beurteilt.
Unter Stabilität ist im Sinne der vorliegenden Erfindung insbesondere zu verstehen, dass auch die treibgasfreie Emulsion vorgenannte Lagertests toleriert, die Emulsion nach Zusatz des Treibgases stabil bleibt bzw. eine gute Aufschüttelbarkeit gewährleistet.

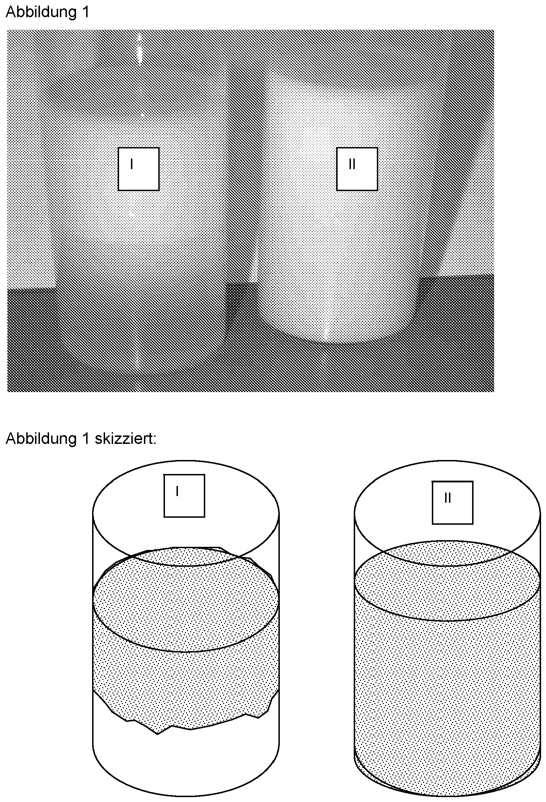

Abbildung 1 zeigt auf der linken Seite (I) eine Emulsion basierend auf einer Emulgatorkombination wie in Druckschrift EP 1 474 099 A1 offenbart in Anlehnung der dortigen Beispiele B1 und B5 unter Zusatz von 10% Aluminum Chlorohydrate (Beispiel E) nach 2 Zyklen im oben beschriebenen Wechseltemperaturtest. Das Muster rechts (II) zeigt die erfindungsgemäße Zubereitung (Beispiel 5) bei gleicher Lagerung.

Besonders bemerkenswert und damit vorteilhaft zeigte sich, dass durch den Zusatz von Guerbet-Alkoholen oder -Ester sich die physikalische Stabilität der erfindungsgemäßen W/O-Emulsionen weiter steigern lässt.

Unter Guerbet-Alkoholen werden verzweigtkettige Alkohole des allgemeinen Schemas 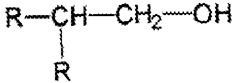 verstanden. Es handelt sich demnach um einen an zwei Positionen verzweigten, einwertigen Alkohol. R stellt dabei bevorzugt Kohlenwasserstoffreste mit C6 bis C12 dar.
Bekannt sind Guerbet Alkohole beispielsweise aus "Lexikon der Hilfsstoffe für Pharmazie, Kosmetik und angrenzende Gebiete", H.P. Fiedler, 3. Aufl., 1989, Editio Cantor Aulendorf. Als besonders bevorzugte Guerbet-Alkohole sind beispielsweise Hexyldecanol, Octyldodecanol und 2- Ethylhexylalkohol zu wählen. Ganz besonders bevorzugt ist Octyldodecanol.
Ferner können auch Guerbet-Alkoholester, sowie Mischungen aus Guerbet-Alkoholen und Guerbet-Alkoholestern (wie z.B. Hexyldecanol und Hexyldecyllaurat) verwendet werden. Durch den Zusatz an Guerbet-Alkoholen bzw. - Estern konnte die physikalische Stabilität der erfindungsgemäßen Emulsion weiter deutlich verbessert werden.
(Der Nachweis der physikalischen Stabilität gegenüber Zubereitungen des Standes der Technik erfolgt üblicherweise visuell und lässt sich graphisch daher nicht darstellen.)

Die erfindungsgemäßen Zubereitungen sind vorteilhaft bei Raumtemperatur dünnflüssig, d.h. mit einer Viskosität von nicht mehr als 1000 mPas, gemessen bei 25°C mit Rheomat R 123, Fa. proRheo, Messkörper 3, und sind sprühfähig, d.h. in gängigen Sprühvorrichtungen, insbesondere Aerosoldosen versprühbar.

Bei der Optimierung der Zubereitung wurde darauf geachtet, dass sich beim Aufsprühen der Zubereitung auf schwarzem Pappkarton ein farbloser, transparenter Film bildet und somit die gestellte Aufgabe der Vermeidung von weißen Flecken (Low-Residue-Eigenschaften) stützt. Es wurde überraschend gefunden, dass die Kombination des Guerbet-Alkohols, insbesondere Octyldodecanol, mit Sorbitanoleaten, insbesondere Sorbitantrioleat, diese Aufgabe am besten löst.
Sorbitantrioleat ist das erfindungsgemäß bevorzugte Oleat, jedoch weisen die anderen Oleate, wie Beispielsweise im Beispiel C gezeigt, einen ebensolchen erfindungsgemäßen Vorteil auf.

Neben den Guerbet-Alkoholen oder - Estern können weitere Lipide der Zubereitung zugesetzt werden. Insbesondere Lipide gewählt aus der Gruppe Cyclomethicone (D4, D5, D6, bevorzugt Cyclopentasiloxane), Dimethicone (bevorzugt 100 cs), Dimethiconol, Phenyltrimethicon, Paraffinum Liquidum, Isopropylpalmitate, C12-15 Alkyl Benzoate, Diethylhexyl Carbonate, Isopropyl Stearate, Dicaprylyl Ether, Persea Gratissima Oil, Cetearyl Ethylhexanoate, Isopropyl Myristate, Caprylic/Capric Trigylceride, Sonnenblumenöl, Glycine Soja (Soybean) Oil, Rapsöl und/oder PPG-14 Butylether.

Dicaprylycarbonat wird erfindungsgemäß bevorzugt als Lipidbestandteil der Zubereitungen ausgeschlossen.
Es hat sich gezeigt, dass die Verwendung von Dicaprylylcarbonat zu weniger stabilen Systemen führt, als im Vergleich zu dem bevorzugten Octyldodecanol. Der Verzicht auf Dicaprylylcarbonat hat sich auch als vorteilhaft bzgl. der Kompatibilität mit polyolefinischen Packmittelkomponenten (z.B. Steigrohr, Ventilinnendichtung, Fingertaste, Sprühkopf bzw. dessen Düse) gezeigt.

Der Anteil der Lipide in der Gesamtformulierung liegt vorteilhaft unterhalb 20 Gew.%, insbesondere unterhalb 15 Gew.%, besonders bevorzugt unterhalb 10 Gew.%, bezogen auf die Gesamtmasse der Zubereitung inklusive Treibgasen.

In weiteren Vergleichsuntersuchungen zeigte sich, dass ein einfacher Austausch des Sorbitanoleats, hier beispielsweise zum Vergleich das Sorbitantrioleat durch Sorbitantristearat (Beispiele A und B), nicht zu den erfindungsgemäßen Verbesserungen führt.
Der Austausch von Sorbitanoleate durch Sorbitantristearat oder Sorbitanmonostearat zeigt (siehe Beispiel A, B und D), dass sich das Sorbitantristearat bzw. Sorbitanmonostearat schlecht einarbeiten lässt, es zu deutlichen Ausfällungen kommt, ein starkes Weißeln im Sprühbild beobachtet wird bzw. die Zubereitung bereits nach 1 Tag bei Raumtemperatur separiert.
Eine einigermaßen zufriedenstellende Einarbeitung der Stearate gelingt nur, wenn der Lipidanteil der Emulsion groß genug gewählt wird, was wiederum der Stabilität und Formulierungsfreiheit sowie Einfachheit, wie erfindungsgemäß gefordert, widerspricht.
Bei einer Reduzierung des Lipidanteils (z.B. auf 25,75% bezogen auf die Gesamtmasse der Zubereitung ohne Treibmittel) separiert die Sorbitantristearat enthaltene Emulsion binnen 24 Stunden, sie ist demnach als wenig stabil zu bezeichnen. Weiterhin ist die Zubereitung mit erhöhtem Lipidgehalt und Sorbitantristearat nicht komplett im Treibmittel löslich und es kommt zu Ausfällungen.

Demgegenüber zeigten die Zubereitungen mit Sorbitanoleaten diese Stabilitätsprobleme nicht, wie Versuche mit Sorbitan-mono-, -di- , -tri- und -tetraoleaten zeigten (siehe z.B. Beispiele 1 - 7 und Beispiel C).

Weitere Vorteile der erfindungsgemäßen Emulgatorkombination ergeben sich beim Einsatz in kosmetischen oder dermatologischen Deodorant- oder Antitranspirantzubereitungen.

Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Die gewünschte Minimierung der Schweißsekretion kann durch verschiedene Mechanismen realisiert werden. Hierzu zählt traditionell der Einsatz von Adstringenzien, welche Eiweißfällungen und Gerinnungen hervorrufen und so eine Verengung oder Verschluss der Schweißdrüse bewirken.

Neuartige AT-Wirker basieren bspw. auf dem Prinzip der Anticholinergika, welche die Nervenreize, die zur Sekretionssteigerung der Schweißdrüsen führen, unterbrechen.

Ein weiteres Prinzip neuartiger AT-Wirker beruht auf der Beeinflussung von Membrantransportvorgängen in der Zelle. So hemmen spezifische Aquaporin-Inhibitoren die Proteine, die Kanäle in der Zellmembran bilden, um den Durchtritt von Wasser und weiteren Molekülen zu erleichtern. Auch lonenkanal-Hemmer bewirken eine Beeinflussung von Membrantransport- bzw. Osmose-Vorgängen. Ein weiterer neuartiger AT-Wirkmechanismus lässt sich durch die Verwendung kurzkettiger, vicinaler Diole realisieren, welcher möglicherweise auf deren osmotische Aktivität zurückzuführen ist.

So durch Anwendung eines Mittels kein Einfluss auf die Schweißsekretion ausgeübt, also keine antitranspirante Wirkung realisiert wird, liegt erfindungsgemäß kein Antitranspirant vor.

Erfindungsgemäß sind demnach als Antitranspirantwirkstoffe solche Stoffe umfasst, die einen Einfluss auf die Schweißsekretion haben.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruches (Geruchsverbesserungsmittel). Gängige Wirkmechanismen hierzu sind antibakterielle Effekte, wie sie auch das nicht-kolloidale Silber zeigen, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen umgesetzt werden.

Zubereitungen können neben den eigentlichen schweißhemmenden Wirkstoffen (AT-Wirker) zusätzlich auch Stoffe enthalten, die den mikrobiellen Abbau des Schweißes hemmen, wie z.B. Triclosan. Triclosan wirkt gegen Gram-positive und Gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

Insbesondere bei Antitranspirantien auf Basis von Aluminiumsalzen kann ggf. auf den weiteren Zusatz von rein desodorierend wirkenden Substanzen verzichtet werden, da diese per se ein antimikrobielles Potential aufweisen. Des Weiteren wird durch das reduzierte Wasserangebot durch die verminderte Schweißsekretion auch das bakterielle Wachstum gehemmt.

Antitranspirantwirkstoffe im Sinne der vorliegenden Anmeldung sind insbesondere aus den folgenden Gruppen zu wählen.

Als Antitranspirantwirkstoffe finden insbesondere Adstringenzien Verwendung, vornehmlich Aluminium-Verbindungen. Die früher eingesetzten, stark sauer wirkenden Salze Aluminiumsulfat oder -chlorid und die Agaricinsäure sind weitgehend durch Aluminiumhydroxychlorid und -alkoholate ersetzt worden. Die nachfolgende Auflistung vorteilhaft einzusetzender Antitranspirant-Wirker soll in keinster Weise einschränkend sein:
Aluminium-Salze:
   - Aluminium-Salze wie Aluminiumchlorid AlCl3, Aluminiumsulfat Al2(SO4)3
   - Aluminiumchloride der empirischen Summenformel [Al2(OH)mCln], wobei m+n=6
   - Aluminiumchlorhydrat [Al2(OH)5Cl] x H2O
      ▪ Standard Al-Komplexe: Locron P (Clariant), Micro-Dry (Reheis), ACH-331 (Summit), Aloxicoll PF 40 (Giulini).
      ∘ Aktivierte Al-Komplexe: Reach 501 (Reheis), AACH-324 (Summit), AACH-7171 (Summit), Aloxicoll P (Giulini), Aloxicoll SD100
   - Aluminiumsesquichlorhydrat [Al2(OH)4,5Cl1,5] x H2O
      ∘ Standard Al-Komplexe: Aluminum Sesquichlorohydrate (Reheis), AACH-308 (Summit)
      ∘ Aktivierte Al-Komplexe: Reach 301 (Reheis)
   - Aluminiumdichlorhydrat [Al2(OH)4Cl2] x H2O
Aluminium-Zirkonium-Salze:
   • Aluminium/Zirkonium Trichlorhydrex Glycin [Al4Zr(OH)13Cl3] x H2O x Gly
      ∘ Standard Al/Zr-Komplexe: Rezal 33GP (Reheis), AZG-7164 (Summit), Zirkonal P3G (Giulini)
      ∘ Aktivierte Al/Zr-Komplexe: Reach AZZ 902 (Reheis), AAZG-7160 (Summit), Zirkonal AP3G (Giulini)
   • Aluminium/Zirkonium Tetrachlorhydrex Glycin [Al4Zr(OH)12Cl4] x H2O x Gly
      ∘ Standard Al/Zr-Komplexe: Rezal 36G (Reheis), AZG-368 (Summit), Zirkonal L435G (Giulini)
      ∘ Aktivierte Al/Zr-Komplexe: Reach 908 (Reheis), AAZG-7167 (Summit), Zirkonal AP4G (Giulini)
   • Aluminium/Zirkonium Pentachlorhydrex Glycin [Al8Zr(OH)23Cl5] x H2O x Gly
   • Aluminium/Zirkonium Octachlorhydrex Glycin [Al8Zr(OH)20Cl8] x H2O x Gly

Ebenso von Vorteil können aber auch Glycin-freie Aluminium/Zirkonium-Salze sein.

Aluminiumsalze können nicht nur als Pulver, sondern auch in gelöster "wässriger" Form eingesetzt werden.

Ebenso kann ein antimikrobieller Silbercitratkomplex, wie er in der DE 20 2008 014 407 U1 beschrieben ist, bevorzugt als kosmetisch wirksamer Bestandteil in den kosmetischen Zubereitungen eingesetzt werden.

Die Antitranspirant-Wirkstoffe aus den zuvor geschilderten Gruppen werden in den erfindungsgemäßen Formulierungen bevorzugt in einer Menge von 1 bis 30 Gew.%, bezogen auf die Gesamtmasse der Zubereitung, d.h. inklusive der ggf. vorhandenen Treibgase, eingesetzt.

Bei Einsatz von z.B. ca. 33,3 Gew.% ACH (Aluminium Chlorohydrate, 50 % aq.) in der Wirkstofflösung für ein Aerosol-Spray (ohne Treibgas) und einem Abfüllverhältnis von etwa 30 : 70 (Wirkstofflösung zu Treibgas) wird ein Anteil von etwa 5 Gew. % ACH im Endprodukt vorhanden sein.

Als Wirkstofflösung wird die Summe aller Bestandteile ohne das Treibgas bezeichnet.

AT-Mittel aus der Gruppe der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid können zu einem Anteil von bevorzugt 0,05 bis 1,0 Gew.%, vorzugsweise 0,1%-0,7%, insbesondere 0,3%-0,5 Gew.%, bezogen auf die Gesamtmasse der Zubereitung zugesetzt werden.

Auch vicinale Diole und ähnliche Wirkstoffe aus der Gruppe der osmotisch aktiven Substanzen können als AT-Wirker den erfindungsgemäßen Zubereitungen zugesetzt werden.

Bevorzugt wird als kosmetisch wirksamer Bestanteil ein oder mehrere Verbindungen aus der Gruppe der Deodorantien, insbesondere Parfüme, Alkohole, antimikrobielle und/oder antitranspirant wirksame Wirkstoffe gewählt.

Selbstverständlich ist es möglich weitere oder andere Antitranspirant Wirkstoffe und/oder Deodorantien als kosmetisch wirksamen Bestandteil zu zusetzen.

Bevorzugt handelt es sich bei den erfindungsgemäßen W/O-Emulsionen um Antitranspirantformulierungen (AT-Zubereitungen).

Nachteilig bei der Verwendung von Antitranspirantien, insbesondere solche enthaltend Aluminiumsalzen, ist, wie zuvor ausgeführt, die Bildung von Rückständen in oder auf der Kleidung, die die Kleidung in unschöner Weise verfärben können, was jedoch erfindungsgemäß vermindert bzw. verhindert wird.

Die erfindungsgemäßen Zubereitungen umfassen bevorzugt Mittel zur Verbesserung der Aufschüttelbarkeit. Die sprühfähig formulierten W/O-Emulsion umfassend ein Agens zur Verbesserung der Aufschüttelbarkeit unter lipophilem Treibgas, wobei das Agens ausgewählt ist aus Estern von mindestens 1-fach ungesättigten Fettsäuren mit mehrwertigen Alkoholen, bevorzugt 4-wertigen Alkoholen, bevorzugt Sorbitan.
Die Fettsäuren werden bevorzugt gewählt aus dem Bereich der Säuren mit C16 bis C22, bevorzugt C18 (Ölsäure).
Ein ganz besonders bevorzugtes Mittel zur Verbesserung der Aufschüttelbarkeit unter lipophilem Treibgas ist Sorbitantrioleat.
Die sprühfähig formulierten W/O-Emulsionen können bevorzugt eine Rohstoffkombination zur Verbesserung der Aufschüttelbarkeit unter lipophilem Treibgas umfassen, wobei die Kombination Guerbet-Alkohole und -Ester von mindestens 1-fach ungesättigten Fettsäuren mit mehrwertigen Alkoholen, bevorzugt 4-wertigen Alkohole, und Fettsäure, bevorzugt C18, umfasst.

Insbesondere ist die Kombination von Octyldodecanol und Sorbitantrioleat bevorzugt.

Die Verwendung der Kombination Guerbet-Alkohole und -Estern von mindestens 1-fach ungesättigten Fettsäuren mit mehrwertigen Alkoholen, bevorzugt 4-wertigen Alkohole, zur Verbesserung der Aufschüttelbarkeit kosmetischer Zubereitungen ist damit prädestiniert.

Die Ester von mindestens 1-fach ungesättigten Fettsäuren mit mehrwertigen Alkoholen, bevorzugt 4-wertigen Alkoholen, bevorzugt Sorbitan, werden erfindungsgemäß zur Verbesserung der Aufschüttelbarkeit von Antitranspirant- oder Deodorantzubereitungen auf Wasser-in-ÖI-Emulsionsbasis unter lipophilem Treibgas umfassend mindestens einen W/O-Emulgator, wobei die Zubereitung frei von Polyglycerin-Emulgatoren ist, verwendet. Bevorzugt werden hierzu Sorbitanoleate verwendet.
Vorteilhaft wird der Zubereitung die Kombination Guerbet-Alkohole und -Ester von mindestens 1-fach ungesättigten Fettsäuren mit mehrwertigen Alkoholen, bevorzugt 4-wertigen Alkohole, und Fettsäure, bevorzugt C18, zugesetzt.

Des Weiteren ist die Verwendung von Sorbitanoleaten zur Reduzierung des Weißeleffektes auf der Haut und/oder Kleidung von Antitranspirant- oder Deodorantzubereitungen auf Wasser-in-Öl-Emulsionsbasis umfassend mindestens einen W/O-Emulgator, wobei die Zubereitung frei von Polyglycerin-Emulgatoren ist, erfindungsgemäß vorteilhaft.

Die erfindungsgemäßen Zubereitungen sind vorteilhaft elektrolythaltig formuliert, d.h. sie umfassen bevorzugt hohe Gehalte an Elektrolyten, wie z.B. Magnesiumsulfat, Natriumchlorid, Kaliumchlorid, Natriumcitrat, basische Al-Chloride. Hoher Gehalt bedeutet ein Gehalt von mehr als 5 Gew.%. Der Gehalt an Elektrolyten wird bevorzugt im Bereich von 5 bis 20 Gew.%, insbesondere 6 bis 10 Gew.%, insbesondere im Bereich von 6,5 bis 8 Gew.%, bezogen auf die Gesamtmasse der Zubereitung ohne Treibmittel, gewählt.

Ganz besonders bevorzugt ist Aluminiumchlorohydrat, in einer Menge von 0,1 - 30 Gew.% eingesetzt, bevorzugt 2 - 6 Gew.%, bezogen auf die Zubereitung ohne Treibmittel.

Der Wassergehalt der erfindungsgemäßen Zubereitungen beträgt mehr als 30 Gew.%, bevorzugt im Bereich von etwa 40 bis 70 Gew.%, insbesondere im Bereich von 41 - 53 Gew.%, bezogen auf die Gesamtmasse der Zubereitung ohne Treibgase.

Den Zubereitungen können weitere Wirkstoffe und Zusätze zugesetzt sein, insbesondere
- Konservierungsmittel und -helfer, wie Phenoxyethanol, Parabene (beispielsweise Methyl-, Ethyl-, Butyl-, Propyl-, Isobutylparaben),
- polare Lösungsmittel, wie Wasser, Feuchthaltemittel, mehrwertige Alkohole, wie Glycerin, Propylene Glycol, Butylene Glycol,
- allgemein Alkandiole, wie sie als Konservierungsmittelhelfer Einsatz finden (z.B. Caprylyl Glycol, Pentylene Glycol) und niedermolekulare PEGs (z.B. PEG-8),
- Stabilisatoren, Komplexbilder und Suspendierhilfen, wie Tocopheryl Acetate, Citric Acid, Talc, Triethanolamine, Trisodium EDTA, Disodium EDTA, BHT, Niacinamide, Magnesium Aluminum Silicate, Sodium Chlorid,
- Parfümöle und deren Riechstoffe, wie beispielsweise Methyl dihydrojasmonate, Methyl 2-Octynoate, Cinnamyl Alcohol, Cinnamal, Isoeugenol, Hydroxycitronellal, Amylcinnamyl Alcohol, Farnesol, Benzyl Alcohol, Anise Alcohol, Eugenol, Benzyl Cinnamate, Phenethyl alcohol, Linalool, Linalyl acetate, 2,6-Dimethyl-7-octen-2-ol, Citral, alpha-Hexylcinnamaldehyde, 2-Acetonapthone-1,2,3,4,5,6,7,8-octahydro-2,3,8,8-tetramethyl, p-t-Butyl-alpha-methyldihydrocinnamic aldehyde, Benzyl acetate, 1,3,4,6,7,8-Hexahydro-4,6,6,7,8,8-hexamethylcyclopenta-gamma-2-benzopyran, Methyl cedryl ketone, Ethylene brassylate, 4-(4-Hydroxy-4-methylpentyl)-3-cyclohexene-1-carboxyaldehyde, Benzyl salicylate, Hexyl salicylate, Orange oil, alpha-Isomethylionone, 4-t-Butylcyclohexyl acetate, Patchouli oil, 3,7-Dimethyl-2,6-octadien-1-ol, Tetrahydrolinalool, Hydroxycitronellal, 3,7-Dimethyl-6-octen-1-ol, Orange terpenes, Heliotropin, Terpinyl acetate, omega-Pentadecalactone, Methyl-alpha-ionone, Lavandin oil, Lemon oil, Bergamot oil, 7-Acetyl-1,1,3,4,4,6-hexamethyltetralin, Coumarin, Ethyllinalool, Amyl salicylate, 2-tert-Pentylcyclohexyl acetate, 3-Methyl-5-phenyl-1-pentanol, Cedrol, Benzyl benzoate, Vanillin, alpha-Amylcinnamaldehyde, d-Limonene, 2-Isobutyl-4-hydroxy-4-methyltetrahydropyran, Terpineol, Lavender oil, Dihydromyrcenol, Citronellal, Pinen-β, Damascenon, Thymol, Citronellol, Geraniol) und deren Lösemittel, wie IPM, DPG, Hecylsalicylat, Triethylcitrat, Acetyltributylcitrat, 1,3-Dimethyl-2-imidazolidinon, sowie sonstige Parfümbestandteile wie z.B. Benzoic Acid, Benzophenone, Benzophenone-3, Benzyl acetate, Benzyl Formate, Benzyl laurate, Benzyl Propionate, Benzyl salicylate, BHA, BHT, Citronellol Iso Valerianate, Citronellyl Acetate, Citronellyl Formate, Ethyl Benzoate, Formaldehyde, Formic Acid, Geranyl Acetate, Geranyl Formate, Isobutyl Benzoate, Linalyl acetate, Linalyl formiate, Methyl Benzoate, Neryl Acetate, Phenoxyethanol, Phenyl Benzoate, Propionic Acid, Terpineol, Triethanolamine, Undecylenic Acid,
- Deo-Wirkstoffe wie Ethylhexylglycerin, Methyl Phenylbutanol, Butyloctanoic Acid, Polyglyceryl-2 Caprate, Octenidine HCL, Silbercitrate,
- sonstige Wirkstoffe wie Panthenol, Bisabolol, Q10 oder Glycerylglucose,
- Extrakte wie z.B. aus/von Cymbopogon Citratus, Persea Gratissima, Pearl, Maris Limus, Ostrea Shell, Fucus Vesiculosus, Hamamelis Virginiana, Chamomilla Recutita, Silk, Cotton Seed Oil, Green Tea Extract (Camelia), Magnolia, Rice, Cashmere, Simmondsia Chinensis (Jojoba) Oil, Cucumber und/oder
- Lösungsvermittler wie z.B. PEG-40 Hydrogenated Castor Oil, Trideceth-9, PPG-1-PEG-9 Lauryl Glycol Ether, PEG-5 Ethylhexanoate, PEG-5 Isononanoate.

Die erfindungsgemäßen Zusammensetzungen können ferner gegebenenfalls in der Kosmetik übliche Zusatzstoffe umfassen, wie beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Verdickungsmittel, anfeuchtende und/oder feucht haltende Substanzen, Fette, Öle, Wachse, sofern nicht ausgeschlossen, oder andere übliche Bestandteile einer kosmetischen oder dermatologischen Formulierung umfassen, wie Emulgatoren, Tenside, Alkohole, Polyole, Polymere, Schaumstabilisatoren, Elektrolyte, organische Lösungsmittel oder Silikonderivate enthalten, sofern sie nicht ausgeschlossen sind bzw. die Stabilitätskriterien erfüllt werden.

Moisturizer können vorteilhaft auch als Antifaltenwirkstoffe zum Schutz vor Hautveränderungen, wie sie z. B. bei der Hautalterung auftreten, verwendet werden.

Erfindungsgemäß sind die weiteren Merkmale als besonders bevorzugte Ausführungsformen vorteilhaft zu wählen.

Die erfindungsgemäßen Zubereitungen besitzen bei Raumtemperatur eine Fließfähigkeit, d.h. die erfindungsgemäßen Zubereitungen werden bevorzugt dünnflüssig und sprühbar formuliert, damit sie u.a. vorteilhaft als Aerosol applizierbar sind.
Vorteilhaft ist die Zubereitung mischbar mit lipophilen Treibmitteln. Die Treibgase werden bevorzugt aus einer Mischung von Butan/Isobutan/Propan, bevorzugt im Verhältnis 3/1/1, wie zum Beispiel ca. 61% n-Butan, ca. 21% i-Butan, ca. 18% Propan, gewählt.

Die erfindungsgemäßen Zubereitungen werden vorteilhaft unter lipophilen Treibmitteln in Druckgasverpackungen versehen. Die Abfüllverhältnisse liegen im Bereich von 10 (Wirkstofflösung) : 90 (Gas) bis 50 (Wirkstofflösung) : 50 (Gas). Besonders bevorzugt ist eine Abfüllung im Verhältnis 30 (Wirkstofflösung) : 70 (Gas).

Die Zubereitungen sind weiterhin vorteilhaft nicht transparent gestaltet (siehe z.B. Abbildung 1) und unterscheiden sich dadurch von transparenten W/Si-Gelen. Vorteilhaft ist, dass auf technologisch aufwändige Verfahren wie die Angleichung von Brechungsindizes oder das Vorliegen feinstdisperser Systeme somit verzichtet werden kann. Zur Erzielung feinstdisperser Systeme sind entweder thermische Verfahren (PIT), mechanische Verfahren (Hochdruckhomogenisierung) oder eine Formulierung im galenisch eng begrenzten Bereich der Mikroemulsionen notwendig, was i.d.R. höhere Einsatzkonzentrationen von Emulgatoren erfordert.

Die Zubereitungen sind frei von flüchtigen Alkoholen, wie Ethanol und Isopropanol, da durch deren Zugabe die physikalische Stabilität und die Aufschüttelbarkeit im Aersosol verschlechtert wird.

Die Zubereitungen sind vorteilhaft frei von Polymeren wie z.B. Acrylaten, Carbomeren, Cellulose-Derivaten, Polyvinylalkoholen, PVP um die rheologischen Eigenschaften im Sinne der Sprühbarkeit zu gewährleisten.

Die Zubereitungen sind vorteilhaft frei von unlöslichen Partikeln, um Verstopfungen zu vermeiden und die Low-Residue Eigenschaft zu unterstützen.

Die erfindungsgemäßen Zubereitungen lassen sich vorteilhaft in folgenden drei beispielhaft dargestellten Herstellverfahren produzieren.

Die Fettphase beinhaltet die Emulgatoren, Lipide und die Mittel zur Verbesserung der Aufschüttelbarkeit.

Die Wasserphase beinhaltet Wasser, AT-Mittel und Konservierungshelfer (z.B. Phenoxyethanol).

### Heiß/heiß-Verfahren:

Hierbei werden beide Phasen auf 65°C erhitzt und direkt nach Phasenvereinigung homogenisiert. Für eine schnellere Emulsionsausbildung ist es von Vorteil die Fettphase vorzulegen.

### Heiß/kalt-Verfahren:

Hierbei wird die Fettphase auf 65°C erhitzt und die Wasserphase kalt verrührt. Die Emulsion lässt sich nur herstellen, wenn die Fettphase vorgelegt wird. Bei Vorlage der Wasserphase findet keine Emulsionsausbildung statt. Die im Heiß/kalt-Verfahren hergestellten Rezepturen zeigen in der Langzeitstabilität und auch bei Wechseltemperaturlagerung sehr gute Ergebnisse. Diese Herstellart ist daher als besonders bevorzugt auszuwählen.

### Kalt/kalt-Verfahren:

Hierbei werden beide Phasen einzeln kalt homogen vermischt und danach unter Homogenisieren vereint. Bei dieser Herstellart ist es ebenfalls vorteilhaft die Fettphase vorzulegen. Allerdings lassen sich die kalt/kalt hergestellten Emulsionen etwas schlechter unter Treibmittel aufschütteln. Der Stabilitätstest dieser Emulsionen zeigt insbesondere bei Wechseltemperaturlagerung etwas schlechtere Ergebnisse auf, als die heiß/heiß und heiß/kalt produzierten Formeln. Daher ist eine heiß/heiß- oder heiß/kalt-Herstellung zu bevorzugen.

Bevorzugte Applikationsform der erfindungsgemäßen Zubereitung ist die Aerosolform. Als Treibmittel kommen bevorzugt verflüssigbare Kohlenwasserstoffe wie Propan, n-Butan, iso-Butan, n-Pentan, iso-Pentan zum Einsatz. Besonders bevorzugt sind Propan, Butan, iso-Butan bzw. Mischungen dieser Treibgase.
Die genannten Gase können im Sinne der vorliegenden Erfindung jeweils einzeln oder in beliebigen Mischungen zueinander verwendet werden. Bevorzugt werden Mischungen mit Druckstufen von 2,0 bis 3,5 bar eingesetzt. Ganz besonders bevorzugt ist eine Druckstufe von 2,7 bar.
Auch polare bzw. hydrophile Treibgase, wie z. B. DME oder Kohlendioxid, können vorteilhaft im Sinne der vorliegenden Erfindung eingesetzt werden, sofern der Anteil an hydrophilen Gasen so gering gewählt wird und lipophiles Treibgas (z. B. Propan/Butan) im Überschuss vorliegt.

Als Druckgasbehälter kommen bekannte Gefäße aus Metall (Aluminium, Weißblech), geschütztem bzw. nicht-splitterndem und splitterndem Glas oder Kunststoff in Frage, bei deren Auswahl Druck- und Bruchfestigkeit, Korrosionsbeständigkeit, leichte Füllbarkeit als auch ästhetische Gesichtspunkte, Handlichkeit, Bedruckbarkeit etc. eine Rolle spielen.

Die Zubereitung kann insbesondere in Alumonoblock- oder Weißblechdosen in den Verkehr gebracht werden.
Vorteilhaft sind diese Dosen mit folgenden Innenschutzlacken beschichtet, die eine gute Beständigkeit gegenüber kosmetischen Zubereitungen aufweisen: Epoxy-Phenol Lacke (z.B. Hoba 7407 P), PAM-Lacke (Polyamidimid), Micoflexlacke (Vinyl-Organosol), Pulverlacke, wie Epoxidharz-, Epoxidharz/Polyester-, Polyester-, Polyurethan- bzw. Acrylat-Pulverlacke.
Die Ventile der Aerosolformen können vorteilhaft Ventilteller aus Aluminium oder Weißblech enthalten, die ebenfalls mit den o.g. Lacken beschichtet sind.

Die erfindungsgemäßen Zubereitungen, insbesondere Deodorant- oder Antitranspirantzubereitungen, verfügen über eine gute Sprühfähigkeit, ermöglichen eine unproblematische Abfüllung als Aerosol, weisen eine gute Kompatibilität mit lipophilen Treibgasen auf und zeigen eine gute Kompatibilität und Stabilität mit Antitranspirantwirkstoffen.

Insbesondere werden somit sprühbare Antitranspirantzubereitungen zur Verfügung gestellt, die eine Verbesserung der Aufschüttelbarkeit der Gesamtzubereitung ermöglichen.

Nachfolgende Beispiele illustrieren die erfindungsgemäßen Zubereitungen. Die darin aufgeführten Mengen sind Gewichtsanteile, bezogen auf die Gesamtmasse der Zubereitung ohne Treibgas. Alle hier aufgeführten Emulsionen wurden im heiß/heiß Verfahren hergestellt.

### Beispiele

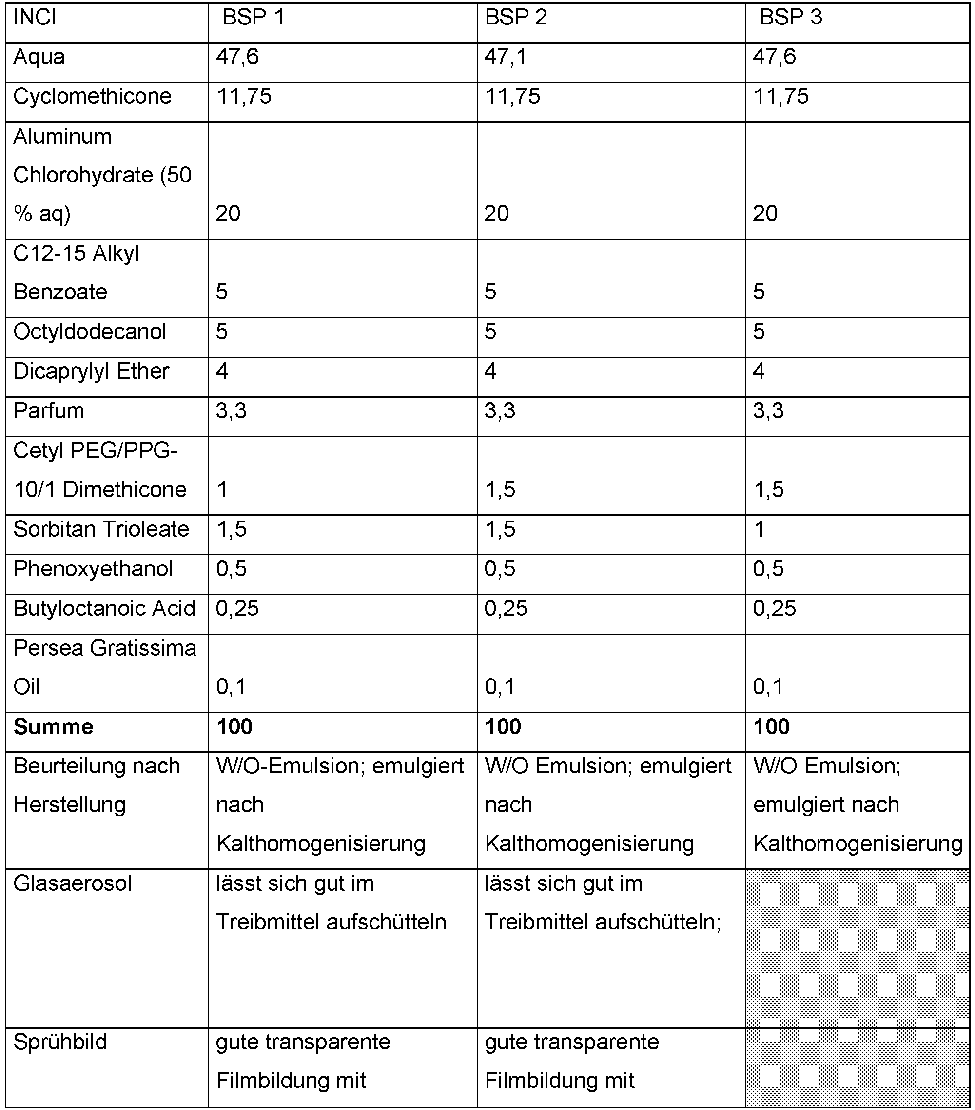

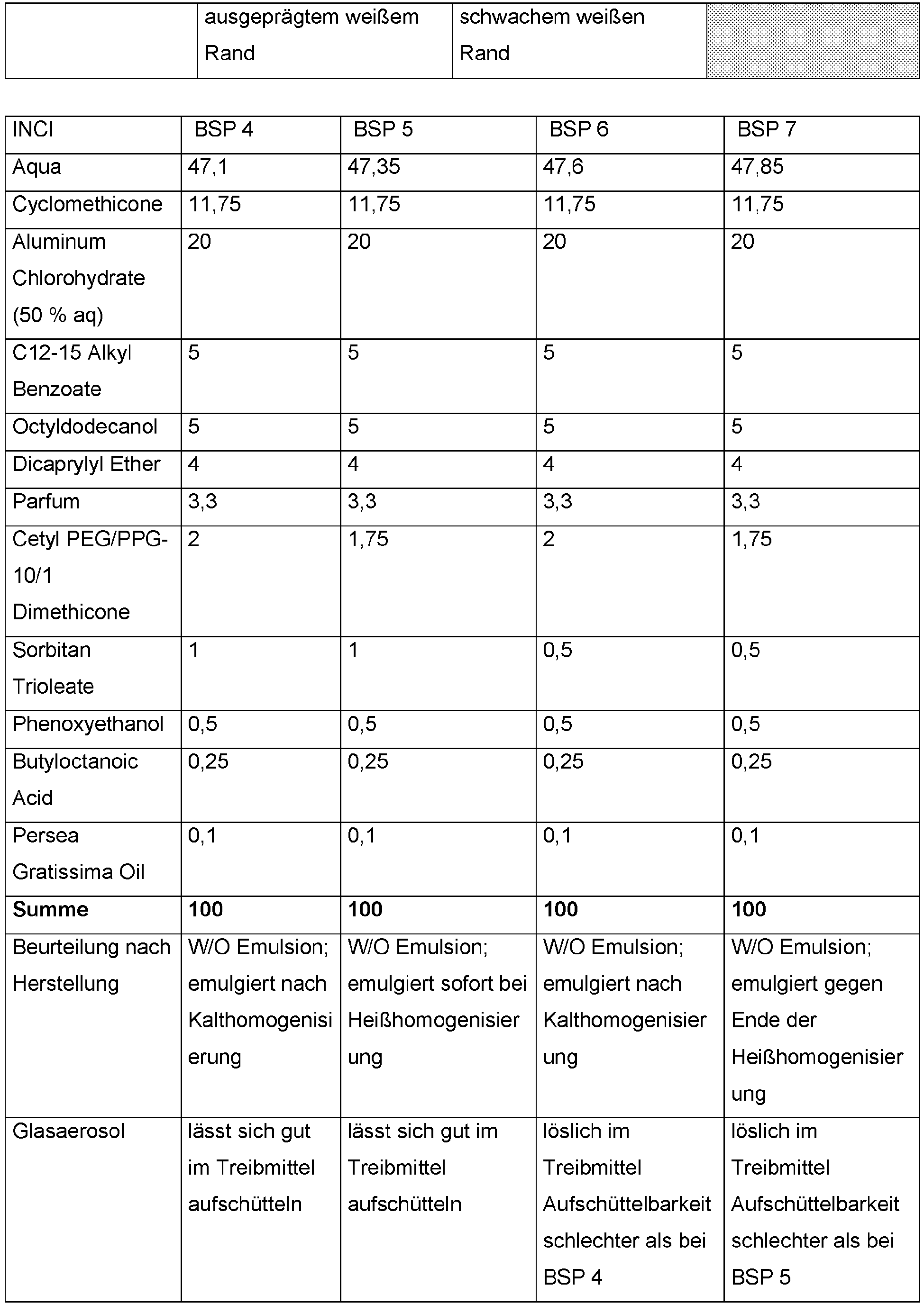

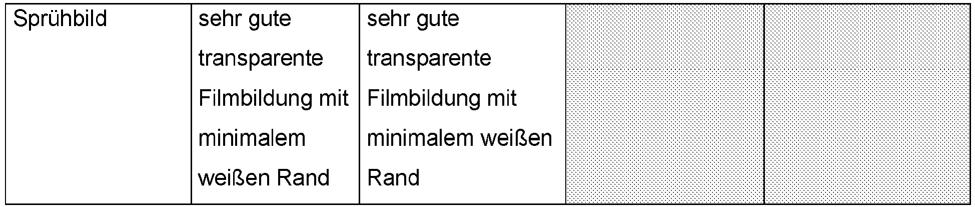

### Beispiele (Vergleichsversuche)

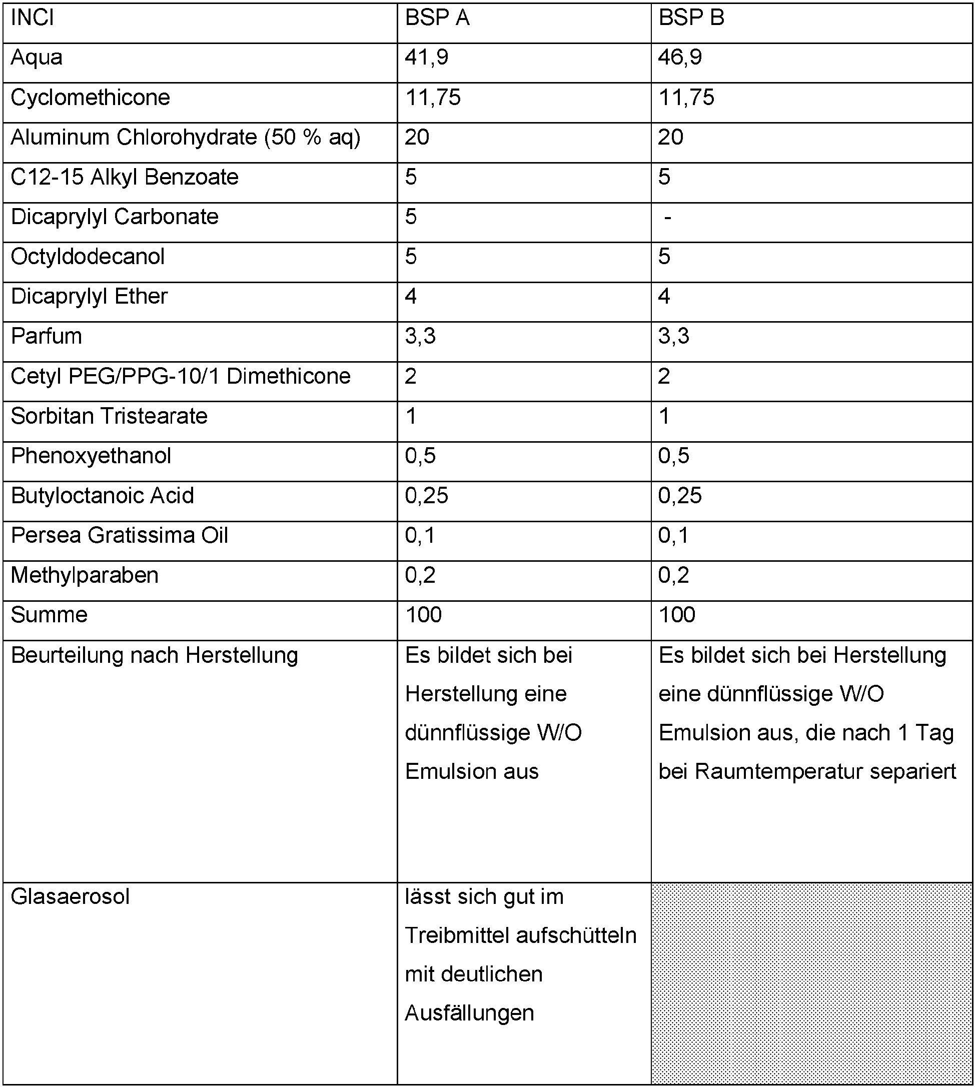

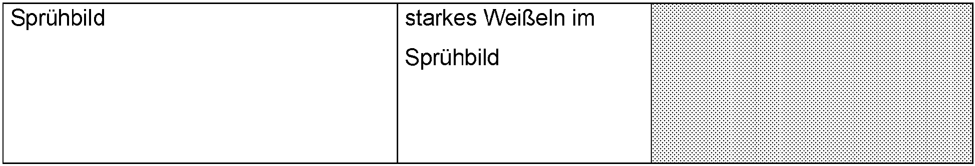

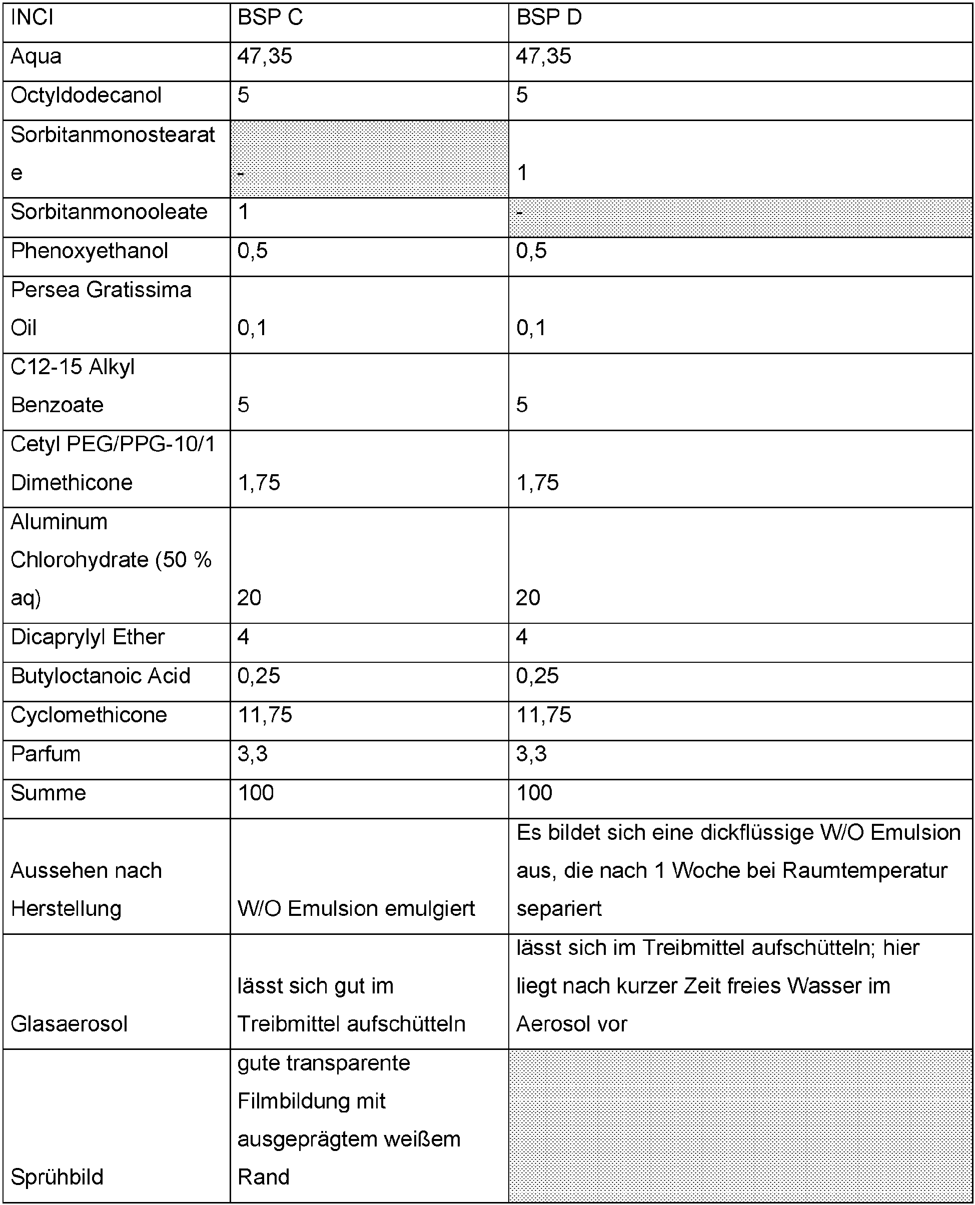

| INCI | BSP E |
|---|---|
| Aqua | 45, 90 |
| Polysorbate-65 | 1 |
| Polyglyceryl-2 Dipolyhydroxystearate | 1,5 |
| Dicaprylyl Carbonate | 5 |
| Phenoxyethanol | 0,5 |
| Methylparaben | 0,2 |
| Persea Gratissima Oil | 0,1 |
| C12-15 Alkyl Benzoate | 5 |
| Cetyl PEG/PPG-10/1 Dimethicone | 1,50 |
| Aluminum Chlorohydrate (50 % aq) | 20 |
| Dicaprylyl Ether | 4 |
| Butyloctanoic Acid | 0,25 |
| Cyclomethicone | 11,75 |
| Parfum | 3,3 |
| Summe | 100 |

Versuche mit den bevorzugten W/O-Emulgatoren, gewählt aus der Gruppe PEG/PPG-18/18 Dimethicone, Lauryl PEG/PPG-18/18 Dimethicone, Bis PEG/PPG 14/14 Dimethicone, Cocamidopropyl PG-Dimonium Chloride, zeigen eine ebensolche Stabilität und Sprüheigenschaften, wie die dargestellten W/O-Emulsionen mit Cetyl PEG/PPG-10/1 Dimethicone.

**Liste benannter und verwendeter Rohstoffe**

| **INCI** | **Handelsname** | **Hersteller/Lieferant** |
|---|---|---|
| | | |
| Aluminum Chlorohydrate (50 % aq) | Chlorhydrol 50 | Summit Reheis Labs |
| Butyloctanoic Acid | Isocarb 12 | Sasol |
| C12-15 Alkyl Benzoate | Tegosoft TN | Evonik |
| Cetyl PEG/PPG-10/1 Dimethicone | Abil EM 90 | Evonik |
| Cyclomethicone | Baysilone SF 1202 | Momentive Performance Materials Inc. |
| Dicaprylyl Carbonate | Cetiol CC | Cognis |
| Dicaprylyl Ether | Cetiol OE | Cognis |
| Methylparaben | Solbrol M | Lanxess |
| Octyldodecanol | Eutanol G | Cognis |
| Persea Gratissima Oil | Avocado Oil, raff.DAC | Henry Lamotte |
| Phenoxyethanol | S&M Phenoxyethanol | Schülke & Mayr |
| Sorbitan Oleate | Span 80 V | Croda |
| Sorbitan Stearate | Arlacel 60 V | Croda |
| Sorbitan Trioleate | Tego STO V | Evonik |
| Sorbitan Tristearate | Nikkol SS-30 | Nikko Chemicals |
| Polysorbate-65 | Tween 65-SO-(MV) | Croda |
| Polysorbate-20 | Tego SML 20 | Evonik |
| Sorbitan Monolaurat | Span 20 | Croda |
| Polyglyceryl-2 Dipolyhydroxystearate | Dehymuls PGPH | Cognis |
| PEG/PPG-18/18 Dimethicone | Dow Corning 190 Fluid | Dow Corning |
| Lauryl PEG/PPG-18/18 Dimethicone | Dow Corning 5200 Formulation Aid | Dow Corning |
| Cocamidopropyl PG-Dimonium Chloride | Lexquat C | Inolex |
| Sorbitan Dioleate | AEC Sorbitan Dioleate | A&E Connock |
| Hexyldecanol | Eutanol G 16 | Cognis |
| Hexyldecyl Laurate | Cetiol PGL | Cognis |

## Patentansprüche

1. Kosmetische oder dermatologische Antitranspirantzubereitung basierend auf einer Wasser-in-ÖI Emulsion umfassend
a. mindestens einen W/O-Emulgator und zusätzlich
b. ein oder mehrere Sorbitan-mono-, -di-, -tri- und/oder -tetraoleate,
c. wobei die Zubereitung einen Wassergehalt von mehr als 30 Gew.% umfasst, bezogen auf die Gesamtmasse der Zubereitung ohne Treibmittel, und
die Zubereitung frei von
d. Polyglycerin-Emulgatoren,
e. Organopolysiloxan-Elastomeren und
f. Ethanol ist
**dadurch gekennzeichnet, dass** die Zubereitung als Aerosol formuliert ist.

2. Zubereitung nach Anspruch 1 **dadurch gekennzeichnet, dass** die Zubereitung Sorbitantrioleate umfasst.

3. Zubereitung nach Anspruch 1 oder 2 **dadurch gekennzeichnet, dass** der oder die Sorbitanoleate zu einem Anteil von bis zu 4 Gew.%, insbesondere bis zu 2 Gew.%, bezogen auf die Gesamtmasse der Zubereitung ohne Treibmittel, gewählt werden.

4. Zubereitung nach einem der vorstehenden Ansprüche **dadurch gekennzeichnet, dass** der oder die W/O-Emulgatoren aus der Gruppe der Emulgatoren mit einem HLB von gleich oder kleiner 5 gewählt werden.

5. Zubereitung nach einem der vorstehenden Ansprüche **dadurch gekennzeichnet, dass** der oder die W/O-Emulgatoren aus der Gruppe PEG/PPG-18/18 Dimethicone, Lauryl PEG/PPG-18/18 Dimethicone, Bis PEG/PPG 14/14 Dimethicone, Cetyl PEG/PPG-10/1 Dimethicone und Cocamidopropyl PG-Dimonium Chloride gewählt werden.

6. Zubereitung nach einem der vorstehenden Ansprüche **dadurch gekennzeichnet, dass** die W/O-Emulgatoren zu einem Anteil von bis zu 5 Gew.%, insbesondere bis zu 2,5 Gew.%, bezogen auf die Gesamtmasse der Zubereitung ohne Treibmittel, gewählt werden.

7. Zubereitung nach einem der vorstehenden Ansprüche **dadurch gekennzeichnet, dass** das Gewichtsverhältnis von W/O-Emulgator zu Sorbitanoleaten im Bereich von 1 bis 4 zu 1 bis 4, insbesondere 1 bis 2 zu 1 bis 1,5 gewählt wird.

8. Zubereitung nach einem der vorstehenden Ansprüche **dadurch gekennzeichnet, dass** die Zubereitung frei von weiteren Emulgatoren ist.

9. Zubereitung nach einem der vorstehenden Ansprüche **dadurch gekennzeichnet, dass** die Zubereitung ein oder mehrere Guerbet-Alkohole oder -Ester, insbesondere Hexyldecanol, Octyldodecanol und/oder 2- Ethylhexylalkohol umfasst.

10. Zubereitung nach Anspruch 9 **dadurch gekennzeichnet, dass** Octyldodecanol der Zubereitung zugesetzt ist.

11. Zubereitung nach einem der vorstehenden Ansprüche **dadurch gekennzeichnet, dass** der Anteil an ethoxilierten Sorbitanfettsäureester in der Zubereitung weniger als 0,25 Gew.%, bevorzugt 0 Gew.%, bezogen auf die Gesamtmasse der Zubereitung ohne Treibgase, umfasst.

12. Zubereitung nach einem der vorstehenden Ansprüche **dadurch gekennzeichnet, dass** der Anteil an Lipiden in der Gesamtformulierung unterhalb 20 Gew.%, insbesondere unterhalb 15 Gew.%, besonders bevorzugt unterhalb 10 Gew.%, bezogen auf die Gesamtmasse der Zubereitung inklusive Treibgasen, liegt.

13. Zubereitung nach einem der vorstehenden Ansprüche **dadurch gekennzeichnet, dass** die Zubereitung frei von Dicaprylycarbonat ist.

14. Zubereitung nach einem der vorstehenden Ansprüche **dadurch gekennzeichnet, dass** die Zubereitung einen Gehalt an Elektrolyten im Bereich von 5 bis 20 Gew.%, insbesondere 6 bis 10 Gew.%, bezogen auf die Gesamtmasse der Zubereitung ohne Treibmittel, umfasst.

15. Zubereitung nach Anspruch 14, **dadurch gekennzeichnet dass** die Zubereitung einen Gehalt an Elektrolyten im Bereich von 6,5 bis 8 Gew.%, bezogen auf die Gesamtmasse der Zubereitung ohne Treibmittel, umfasst.

16. Zubereitung nach einem der vorstehenden Ansprüche **dadurch gekennzeichnet, dass** als Treibgas lipophile Treibmittel gewählt werden.

17. Zubereitung nach Anspruch 16 **dadurch gekennzeichnet, dass** als lipophile Treibmittel Propan, Butan und/oder dessen Isomere und/oder beliebige Mischungen davon gewählt werden.

18. Zubereitung nach einem der Ansprüche 16 oder 17 **dadurch gekennzeichnet, dass** die Treibgase aus einer Mischung von Butan/Isobutan/Propan, bevorzugt im Verhältnis 3/1/1, gewählt werden.

19. Zubereitung nach Anspruch 17 oder 18 **dadurch gekennzeichnet, dass** Treibmittel mit Druckstufen von 2,0 bis 3,5 bar, insbesondere mit einer Druckstufe von 2,7 bar, gewählt werden.

## Claims

1. Cosmetic or dermatological antiperspirant preparation based on a water-in-oil emulsion comprising
a. at least one W/O emulsifier and additionally
b. one or more sorbitan mono-, di-, tri and/or tetraoleates,
c. wherein the preparation comprises a water content of more than 30% by weight,
based on the total weight of the preparation without propellant, and
the preparation is free of
d. polyglycerol emulsifiers
e. organopolysiloxane elastomers and
f. ethanol
**characterized in that** the preparation is formulated as an aerosol.

2. Preparation according to Claim 1, **characterized in that** the preparation comprises sorbitan trioleate.

3. Preparation according to Claim 1 or 2, **characterized in that** the sorbitan oleate(s) is/are selected at a proportion of up to 4% by weight, especially up to 2% by weight, based on the total mass of the preparation without propellant.

4. Preparation according to any of the preceding claims, characterized in-that the W/O emulsifier(s) is/are selected from the group of emulsifiers having an HLB of equal to or less than 5'.

5. Preparation according to any of the preceding claims, **characterized in that** the W/O emulsifier(s) is/are selected from the group comprising PEG/PPG-. 18/18 dimethicone, lauryl PEG/PPG-18/18 dimethicone, bis PEG/PPG-14/14 dimethicone, cetyl PEG/PPG-10/1 dimethicone and cocamidopropyl PG-dimonium chloride.

6. Preparation according to any of the preceding claims, **characterized in that** the W/O emulsifiers are selected at a proportion of up to 5% by weight, especially up to 2.5% by weight, based on the total mass of the preparation without propellant.

7. Preparation according to any of the preceding claims, **characterized in that** the ratio by weight of W/O emulsifier to sorbitan oleates is selected in the range from 1-4 : 1-4, especially from 1-2 : 1-1.5.

8. Preparation according to any of the preceding claims, **characterized in that** the preparation is free of further emulsifiers.

9. Preparation according to any of the preceding claims, **characterized in that** the preparation comprises one or more Guerbet alcohols or esters, especially hexyldecanol, octyldodecanol and/or 2-ethylhexanol.

10. Preparation according to Claim 9, **characterized in that** octyldodecanol is added to the preparation.

11. Preparation according to any of the preceding claims, **characterized in that** the proportion of ethoxylated sorbitan fatty acid esters in the preparation comprises less than 0.25% by weight, preferably 0% by weight, based on the total mass of the preparation without propellant gas.

12. Preparation according to any of the preceding claims, **characterized in that** the proportion of lipids in the total formulation is below 20% by weight, particularly below 15% by weight, particularly preferably below 10% by weight, based on the total mass of the preparation including propellant gases.

13. Preparation according to any of the preceding claims, **characterized in that** the preparation is free of dicaprylyl carbonate.

14. Preparation according to any of the preceding claims, **characterized in that** the preparation comprises a content of electrolytes in the range of 5 to 20% by weight, especially 6 to 10% by weight, based on the total mass of the preparation without propellant.

15. Preparation according to Claim 14, **characterized in that** the preparation comprises a content of electrolytes in the range of 6.5 to 8% by weight, based on the total mass of the preparation without propellant.

16. Preparation according to any of the preceding claims, **characterized in that** lipophilic propellants are selected as propellant gas.

17. Preparation according to Claim 16, **characterized in that** the lipophilic propellants selected are propane, butane and/or isomers thereof and/or any mixtures thereof.

18. Preparation according to either of Claims 16 or 17, **characterized in that** the propellant gases are selected from a mixture of butane/isobutane/propane, preferably in the ratio 3/1/1.

19. Preparation according to Claim 17 or 18, **characterized in that** propellants are selected having pressure levels of 2.0 to 3.5 bar, especially having a pressure level of 2.7 bar.

## Revendications

1. Préparation antitranspirante cosmétique ou dermatologique à base d'une émulsion eau-dans-huile comprenant
a. au moins un émulsifiant E/H (eau-dans-huile) et de plus
b. un ou plusieurs monooléate(s), dioléate(s), trioléate(s) et/ou tétraolate(s) de sorbitane,
c. la préparation comprenant une teneur en eau supérieure à 30% en poids, par rapport à la masse totale de la préparation sans agent propulseur et
la préparation étant exempte
d. d'émulsifiants de type polyglycérol,
e. d'élastomères de type organopolysiloxane et
f. d'éthanol
est **caractérisée en ce que** la préparation est formulée sous forme d'aérosol.

2. Préparation selon la revendication 1, **caractérisée en ce que** la préparation comprend des trioléates de sorbitane.

3. Préparation selon la revendication 1 ou 2, **caractérisée en ce que** l'oléate ou les oléates de sorbitane est/sont choisi(s) en une proportion de jusqu'à 4% en poids, en particulier jusqu'à 2% en poids, par rapport à la masse totale de la préparation sans agent propulseur.

4. Préparation selon l'une quelconque des revendications précédentes, **caractérisée en ce que** l'émulsifiant ou les émulsifiants E/H est/sont choisi(s) dans le groupe des émulsifiants présentant une valeur de HLB (Hydrophile/Lipophile Balance - équilibre hydrophile/lipophile) inférieure ou égale à 5.

5. Préparation selon l'une quelconque des revendications précédentes, **caractérisée en ce que** l'émulsifiant ou les émulsifiants E/H est/sont choisi(s) dans le groupe du PEG/PPG-18/18 diméthicone, du lauryl-PEG/PPG-18/18 diméthicone, du bis-PEG/PPG 14/14 diméthicone, du cétyl-PEG/PPG-10/1 diméthicone et du chlorure de cocamidopropyl PG-dimonium.

6. Préparation selon l'une quelconque des revendications précédentes, **caractérisée en ce que** les émulsifiants E/H sont choisis en une proportion de jusqu'à 5% en poids, en particulier jusqu'à 2,5% en poids, par rapport à la masse totale de la préparation sans agent propulseur.

7. Préparation selon l'une quelconque des revendications précédentes, **caractérisée en ce que** le rapport pondéral de l'émulsifiant E/H aux oléates de sorbitane est choisi dans la plage de 1-4 : 1-4, en particulier dans la plage de 1-2 : 1-1,5.

8. Préparation selon l'une quelconque des revendications précédentes, **caractérisée en ce que**, la préparation est exempte d'autres émulsifiants.

9. Préparation selon l'une quelconque des revendications précédentes, **caractérisée en ce que** la préparation comprend un ou plusieurs alcool(s) ou ester(s) de Guerbet, en particulier de l'hexyldécanol, de l'octyldodécanol et/ou de l'alcool 2-éthylhexylique.

10. Préparation selon la revendication 9, **caractérisée en ce que** de l'octyldodécanol est ajouté à la préparation.

11. Préparation selon l'une quelconque des revendications précédentes, **caractérisée en ce que** la proportion d'ester d'acide gras de sorbitane éthoxylé dans la préparation est inférieure à 0,25% en poids, préférablement est de 0% en poids, par rapport à la masse totale de la préparation sans gaz propulseurs.

12. Préparation selon l'une quelconque des revendications précédentes, **caractérisée en ce que** la proportion de lipides dans la formulation totale se situe en dessous de 20% en poids, en particulier en dessous de 15% en poids, particulièrement préférablement en dessous de 10% en poids, par rapport à la masse totale de la préparation, gaz propulseurs inclus.

13. Préparation selon l'une quelconque des revendications précédentes, **caractérisée en ce que** la préparation est exempte de carbonate de dicaprylyle.

14. Préparation selon l'une quelconque des revendications précédentes, **caractérisée en ce que** la préparation comprend une teneur en électrolytes dans la plage de 5 jusqu'à 20% en poids, en particulier dans la plage de 6 jusqu'à 10% en poids, par rapport à la masse totale de la préparation sans agent propulseur.

15. Préparation selon la revendication 14, **caractérisée en ce que** la préparation comprend une teneur en électrolytes dans la plage de 6,5 jusqu'à 8% en poids, par rapport à la masse totale de la préparation sans agent propulseur.

16. Préparation selon l'une quelconque des revendications précédentes, **caractérisée en ce que** des agents propulseurs lipophiles sont choisis en tant que gaz propulseur.

17. Préparation selon la revendication 16, **caractérisée en ce que** le propane, le butane et/ou ses isomères et/ou des mélanges quelconques correspondants est/sont choisi(s) en tant qu'agents propulseurs lipophiles.

18. Préparation selon la revendication 16 ou 17, **caractérisée en ce que** les gaz propulseurs sont choisis parmi un mélange de butane/isobutane/propane, préférablement selon un rapport 3/1/1.

19. Préparation selon la revendication 17 ou 18, **caractérisée en ce que** des agents propulseurs sont choisis, qui présentent des niveaux de pression de 2,0 jusqu'à 3,5 bars, en particulier qui présentent un niveau de pression de 2,7 bars.
